# EUROPEAN PATENT APPLICATION

(11) **EP 4 578 463 A1**
(43) Date of publication of application: **02.07.2025**
(21) Application number: 23383412.6
(22) Date of filing: 29.12.2023
(51) Int. Cl.: A61K 47/68, A61P 35/00

(54) **ENDO-180 TARGETED ANTIBODY-DRUG CONJUGATES**

(71) Applicant: Oncomatryx Biopharma, S.L., 48160 Derio (Vizcaya) (ES)
(72) Inventor: The designation of the inventor has not yet been filed
(74) Representative: Mewburn Ellis LLP

(57) **Abstract**

The present invention relates to anti-Endo180-targeted antibody-drug conjugates (ADCs), in particular anti-Endo180 antibody-drug conjugates having the formula A-(L-D)p, or a pharmaceutically acceptable salt or solvate thereof, wherein A is an antibody that specifically binds domain III of Endo180, L is a linker, D is a drug comprising a topoisomerase inhibitor or a cytolysin and p is 1 to 20, for use in a method of treatment of cancer, fibrosis or inflammatory diseases in a mammalian subject.

## Description

### Field of the Invention

The present invention relates to antibody-drug conjugates (ADCs) that target Endo180, and to their use in medicine, e.g. in the treatment of certain cancers.

### Background

Monoclonal antibody (MAb) - based drugs show great potential in treating cancer. However, MAbs that target cell surface tumor antigens rarely have sufficient efficacy on their own. To increase their low activity, they can be bound to cytotoxic molecules. The antibody must be very selective to reach the antigen, whose expression must be restricted in normal cells to avoid off-target effects. The antibody also must be internalized efficiently into the cancerous cells to enable the cytotoxic agent to take effect.

The field of Antibody-Drug conjugates (ADCs) for the treatment of cancer has experienced a growing development activity by pharmaceutical companies, due to the technological advances of the last few years, aimed at solving the problems ADCs initially presented, such as undesirable toxicity, production, half-life and resistance.

The cytotoxic agent selected as the effector moiety must kill cells only after internalization and release into the cell cytoplasm in order to maintain specificity. The most commonly used payloads in ADCs are DNA-harming drugs such as calicheamicins, duocarmicins, or microtubule-targeting compounds like auristatins and maitansinoids.

The linkers between the antibody and the cytotoxic agent are designed to be stable systemically and to release the cytotoxic within the target cells.

Endo180 (also known as uPARAP) is a type I transmembrane protein of the mannose receptor family that is encoded by the MRC2 gene. In healthy, adult individuals it has a restricted expression in minor subsets of mesenchymal cells, macrophages, and some epithelial cells. Endo180 functions as a clathrin-dependent endocytic receptor for collagen and other extracellular matrix (ECM) components. It also interacts with urokinase plasminogen activator (uPA), a protease involved in ECM remodeling and cell migration.

Endo180 plays a role in a variety of cellular processes, including:
- ECM remodeling: Endo180 is involved in remodeling the ECM by internalizing and degrading collagen and other ECM components. When collagen binds to Endo180, the receptor is endocytosed into clathrin-coated vesicles and directed to endosome where it dissociates from the receptor. This is important for tissue homeostasis, wound healing, and development. Endo180 may also promote the establishment of a new ECM (Curino, Alejandro C et al.).
- Cell migration: Endo180 would promote cell migration through interaction with uPA and other cell surface receptors, or through activation of signaling pathways such as the MAPK and NF-κB pathways. For example, Endo180 interacts with uPA to promote the cleavage of plasminogen to plasmin. Plasmin is a protease that can degrade collagen and other ECM components, and thus facilitate cell migration (Gucciardo, Fabrice et al.).

Endo180 plays a role in several diseases, including cancer, fibrosis, and inflammatory diseases. In cancer, Endo180 has been shown to be overexpressed in several cancers including sarcomas, glioblastomas and subsets of acute myeloid leukemia. This upregulated expression in cancer is found in cancer-associated fibroblasts (CAFs), and particularly the subset of myofibroblastic CAFs. The collagen receptor may promote tumor growth and metastasis by supporting cell migration and invasion and the production of angiogenic factors required for tumor growth(Gucciardo, Fabrice et al.).

In 2017, Nielsen, Christoffer Fagernaes et al. constructed an Endo180-directed ADC by using a specific monoclonal antibody (2H9) coupled to the dolastatin derivative MMAE. The resulting ADC showed target-dependent cytotoxicity *in vitro* in several non-solid tumor and sarcoma cell lines and was also tested *in vivo* in a murine model transplanted with U937 cells, showing eradication of the tumors (Nielsen, 2017). The same group published 4 years later the expression of Endo180 in malignant mesotheliomas (MM) and proved an *in vitro* cytotoxicity in MM cell lines of an ADC with an anthracycline class cytotoxin, PNU-159682 linked to the antibody 9B7 (Çak 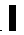 lkaya, 2021).

A couple of years later, Evans et al. also developed an ADC targeting Endo180, where A5/158 antibody was coupled to MMAE payload. This ADC was tested and showed *in vitro* cytotoxicity in multiple sarcoma cell lines, and tumor regression and impairment of metastasis in an *in vivo* sarcoma model (Evans, 2023).

Both ADCs tested in *in vivo* experiments used MMAE as payload, a cytotoxic drug that disrupts microtubule assembly and inhibits mitosis, already used in many other ADCs in development or already approved.

Despite these advances, there remains an unmet need for further therapeutic strategies for the treatment of cancers. The present invention has been devised in light of the above considerations.

### Summary of the Invention

Broadly, the present invention relates to anti-Endo180 antibodies, conjugates thereof and payloads for use in antibody-conjugate strategies. In particular, the present inventors have found that the anti-Endo180 antibodies described herein exhibit highly specific binding to domain III of Endo180, and fast and efficient internalisation.

The topoisomerase inhibitors and cytolysin derivatives described herein are advantageously conjugated to anti-Endo180 antibodies for use in the treatment of cancers.

Accordingly, in a first aspect the present invention provides a conjugate having the formula I:

A - (L-D)ₚ (I)

or a pharmaceutically acceptable salt or solvent thereof,
wherein:
A is an antibody that specifically binds domain III of Endo180;
L is a linker;
D is a drug comprising a topoisomerase inhibitor or a cytolysin; and
p is 1 to 20.

In some cases, p is 1, 2, 3, 4, 5, 6, 7, 8, 9 or 10. In certain cases, p is between 1-5.

### Antibody

In some cases, in accordance with this and other aspects of the present invention, A exhibits a binding affinity dissociation constant K_{D} for domain III of Endo180 of less than 500 nM.

In some cases, A is a monoclonal antibody or binding fragment thereof that specifically binds to domain III of human and/or murine Endo180. In certain cases, human and murine Endo180 consist of the amino acid sequences of SEQ ID NO: 61 and SEQ ID NO: 62 respectively. In certain cases, A binds an epitope located within the region of human Endo180 from S324 to E332. In certain cases, this region consists of the amino acid sequence of SEQ ID NO: 63. Preferably, A binds an epitope located within the amino acid sequence SDQPDNPSE (SEQ ID NO: 63) of domain III of human Endo180.

In some cases, A comprises heavy chain complementarity determining regions 1-3 (CDRH1-3) and light chain complementarity determining regions 1-3 (CDRL1-3) having the following amino acid sequences:
(i) CDRH1: GFTFX₁SYX₂MN;
(ii) CDRH2: NIKPX₃GX₄ERHSVX₅SVKG;
(iii) CDRH3: PGAGRLDY;
(iv) CDRL1: QGDX₆LRX₇X₈X₉AS;
(v) CDRL2: X₁₀X₁₁NX₁₂RPS; and
(vi) CDRL3: NSX₁₃X₁₄X₁₅X₁₆X₁₇X₁₈X₁₉X₂₀A;
wherein:
X₁ is S or P;
X₂ is S or A;
X₃ is D or N;
X₄ is S or N;
X₅ is D or E;
X₆ is S or R;
X₇ is S, R or G;
X₈ is Y, H, N, F or S;
X₉ is Y or F;
X₁₀ is G, F or V;
X₁₁ is K, R, or Q;
X₁₂ is N, K, G or S;
X₁₃ is R, L or P;
X₁₄ is D, G, N, or S;
X₁₅ is S, R, G or T;
X₁₆ is S or G;
X₁₇ is G, T, R or V;
X₁₈ is N, H, T or I;
X₁₉ is P or S; and
X₂₀ is W or P.

In certain cases, CDRH1-3 comprises the following amino acid sequences:
(i) CDRH1: GFTFSSYSMN;
(ii) CDRH2: NIKPDGSERHSVDSVKG; and
(iii) CDRH3: PGAGRLDY.

In certain cases, CDRH1-3 comprises the following amino acid sequences:
(i) CDRH1: GFTFPSYAMN;
(ii) CDRH2: NIKPNGNERHSVESVKG; and
(iii) CDRH3: PGAGRLDY.

In certain cases, CDRL1-3 comprises the following amino acid sequences:
(i) CDRL1: QGDSLRSYYAS;
(ii) CDRL2: GKNNRPS; and
(iii) CDRL3: NSRDSSGNPWA.

In certain cases, CDRL1-3 comprises the following amino acid sequences:
(i) CDRL1: QGDSLRSHYAS;
(ii) CDRL2: FRNKRPS; and
(iii) CDRL3: NSRGSSGNPWA.

In certain cases, CDRL1-3 comprises the following amino acid sequences:
(i) CDRL1: QGDSLRSNFAS;
(ii) CDRL2: VKNGRPS; and
(iii) CDRL3: NSRNSSTHPWA.

In certain cases, CDRL1-3 comprises the following amino acid sequences:
(i) CDRL1: QGDRLRRNYAS;
(ii) CDRL2: GRNKRPS; and
(iii) CDRL3: NSRDRSGNPWA.

In certain cases, CDRL1-3 comprises the following amino acid sequences:
(i) CDRL1: QGDSLRSFYAS;
(ii) CDRL2: GKNKRPS; and
(iii) CDRL3: NSLGSSGNPPA.

In certain cases, CDRL1-3 comprises the following amino acid sequences:
(i) CDRL1: QGDRLRRNYAS;
(ii) CDRL2: GRNNRPS; and
(iii) CDRL3: NSRDGSGTPWA.

In certain cases, CDRL1-3 comprises the following amino acid sequences:
(i) CDRL1: QGDSLRRSYAS;
(ii) CDRL2: FKNNRPS; and
(iii) CDRL3: NSRGTSRNPWA.

In certain cases, CDRL1-3 comprises the following amino acid sequences:
(i) CDRL1: QGDSLRGYYAS;
(ii) CDRL2: GQNKRPS; and
(iii) CDRL3: NSRSRSRIPWA.

In certain cases, CDRL1-3 comprises the following amino acid sequences:
(i) CDRL1: QGDSLRGYYAS;
(ii) CDRL2: GRNSRPS; and
(iii) CDRL3: NSRNRSGHPWA.

In certain cases, CDRL1-3 comprises the following amino acid sequences:
(i) CDRL1: QGDSLRSYYAS;
(ii) CDRL2: FKNNRPS; and
(iii) CDRL3: NSRGSSRNPWA.

In certain cases, CDRL1-3 comprises the following amino acid sequences:
(i) CDRL1: QGDSLRSYFAS;
(ii) CDRL2: FKNNRPS; and
(iii) CDRL3: NSPNSGVISWA.

In certain cases, A comprises a heavy chain variable region (VH) comprising the amino acid sequence of SEQ ID NO: 3.

In certain cases, A comprises a heavy chain variable region (VH) comprising the amino acid sequence of SEQ ID NO: 4.

In certain cases, A comprises a light chain variable region (LH) comprising an amino acid sequence selected from the group consisting of SEQ ID NO: 5-14.

In certain cases, A comprises a light chain variable region (LH) comprising the amino acid sequence of SEQ ID NO: 15.

Preferably, A comprises a heavy chain variable region (VH) comprising the amino acid sequence of SEQ ID NO: 3 and a light chain variable region (LH) comprising an amino acid sequence selected from the group consisting of SEQ ID NO: 5-14 or a heavy chain variable region (VH) comprising the amino acid sequence of SEQ ID NO: 4 and a light chain variable region (LH) comprising the amino acid sequence of SEQ ID NO: 15.

### Topoisomerase inhibitor

In accordance with this and other aspects of the present invention, D may be a topoisomerase inhibitor. In some cases, the topoisomerase inhibitor is of formula II: 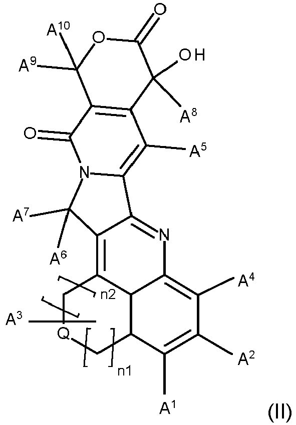 wherein:
A¹ and
A² are each independently selected from the group consisting of halo, hydrogen, C₁-C₃ alkyl, phenyl, hydroxy, C₁-C₃ alkoxy,
or wherein A¹ and A² together with the atoms to which they are bonded form a fused ring;
Q is O, S or CR^{a}R^{b}; wherein R^{a} and R^{b} are each independently selected from hydrogen or a C₁-C₃ alkyl;
n₁ and n₂ are each individually 0, 1 or 2;
A³ is 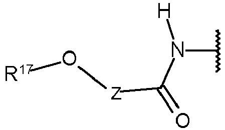 wherein
   Z is a C₁-C₆ alkyl chain or a substituted or unsubstituted aryl group or wherein Z is X₁-Ar wherein X₁ is a C₁-C₆ alkyl chain and Ar is a substituted or unsubstituted aryl group;
   A⁴, A⁵, A⁶ A⁷, A⁹ and A¹⁰ are each individually hydrogen or a C₁-C₃ alkyl;
   A⁸ is hydrogen or a C₁-C₄ alkyl;
   R¹⁷ is indirectly or directly attached to linker L;
   wherein A³ represents a group which may be attached at any point on the ring structure containing Q.

Preferably, Z is a C₁-C₆ alkyl chain. Preferably, Z is a linear C₁-C₆ alkyl chain. Preferably, wherein Q is O, S or CR^{a}R^{b} and wherein R^{a} and R^{b} are each independently selected from hydrogen or a linear C₁-C₃ alkyl chain.

Preferably, wherein A¹ and A² are each independently selected from the group of halo, hydrogen, a linear C₁-C₃ alkyl chain, phenyl, hydroxy, C₁-C₃ alkoxy, or wherein A¹ and A² together with the atoms to which they are bonded form a fused ring.

The ring structure containing substituent Q may be 4, 5, 6, 7 or 8 atoms in size depending on the values of n₁ and n₂. Preferably, the ring containing substituent Q is 5, 6, 7 or 8 atoms in size.

In some cases, in accordance with the first aspect of the present invention, the topoisomerase inhibitor is of formula III: 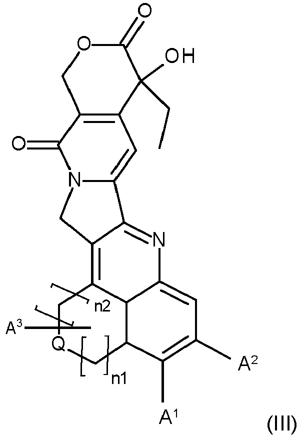 wherein:
A¹ is a C₁-C₃ alkyl chain;
A² is halo;
Q is O, S or CH₂
n₁ and n₂ are each individually 0, 1 or 2;
A³ is 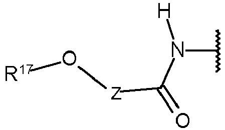 wherein
   Z is a C₁-C₃ alkyl chain and R¹⁷ is indirectly or directly attached to linker L. Preferably, R¹⁷ is indirectly attached to the linker.

Preferably, A¹ is a linear C₁-C₃ alkyl chain. Preferably, Z is a linear C₁-C₃ alkyl chain.

In certain cases, the topoisomerase inhibitor is preferably (1S,9S)-1-Amino-9-ethyl-5-fluoro-9-hydroxy-4-methyl-1,2,3,9,12,15-hexahydro-10H,13H-benzo[de]pyrano[3',4':6,7]indolizino[1,2-b]quinoline-10,13-dione, which corresponds to a compound according to formula IV: 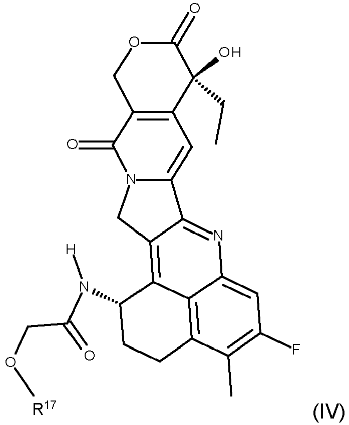 wherein:
R¹⁷ is indirectly or directly attached to linker L. Preferably, R¹⁷ is indirectly attached to linker L.

### Cytolysin

In accordance with this and other aspects of the present invention, D may be a cytolysin. In some cases, the cytolysin is of formula V: 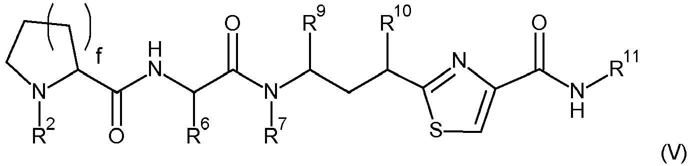 wherein:
R² is H or C₁-C₄ alkyl;
R⁶ is C₁-C₆ alkyl;
R⁷ is C₁-C₆ alkyl, CH₂OR¹⁹ or CH₂OCOR²⁰, wherein R¹⁹ is alkyl, R²⁰ is C₂-C₆-alkenyl, phenyl, or CH₂-phenyl;
R⁹ is C₁-C₆ alkyl;
R¹⁰ is O-alkyl, H, OH, or O-acetyl;
f is 2 or 1;
R¹¹ has the following structure: 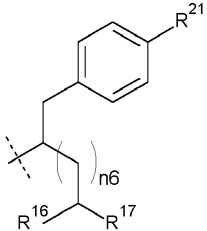 wherein
   R²¹ is OH, H, halogen, NH₂, alkyloxy, phenyl, alkyl amino or dialkyl amino;
   R¹⁶ is a C₁-C₆-alkyl or H;
   R¹⁷ is indirectly or directly attached to linker L; and
   n6 is 0, 1, 2 or 3;
   wherein 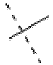 indicates the point of attachment to formula (V) and R¹¹.

In certain cases, the cytolysin is preferably of formula VI 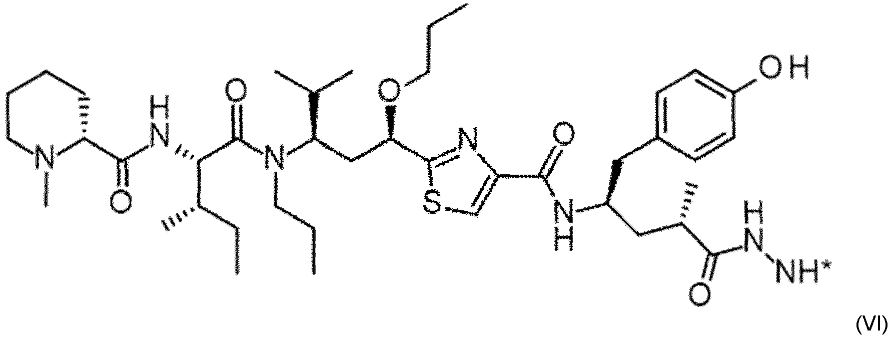 wherein * indicates the site of attachment to L.

### Linker

In some cases, including when D is a topoisomerase inhibitor or a cytolysin, R¹⁷ is C(O)X, CONHNHX, OX, NHX or SX, wherein X is a bond to linker L.

In some cases, the linker L further comprises a spacer. In some cases, the spacer has a chain length of 2 to 30 atoms. In some cases, the spacer comprises or consists of an oxyalkylene or alkylene group. In some cases, the spacer comprises or consists of a group -(OCH₂CH₂)ₙ- or -(CH₂)ₙ-, wherein n ≥ 1. In some cases, n = 1 to 15, 1 to 10, 2 to 9, 1 to 6, 2 to 5 or 3 to 5. In certain cases, n = 3 or 4. In certain cases, n = 1, n = 2, n = 3, n = 4, n = 5, n = 6, n = 7, n = 8, n=9 or n = 10.

In some cases, the spacer is attached to group R¹⁷ via a bridging group or is directly attached to group R¹⁷. In some cases, the spacer is attached to group R¹⁷ via a -C(O)X bridging group, wherein X is a bond to R¹⁷. In some cases, R¹⁷ is CONHNHX and the spacer is attached to group R¹⁷ via a -C(O)X bridging group, wherein X represents the bond between the spacer and R¹⁷. Preferably, R¹⁷ is CONHNHX and the spacer is a -(OCH₂CH₂)ₙ- attached to R¹⁷ via a -C(O)X bridging group, wherein n = 2, 3 or 4. In some places in this application R¹⁷ is depicted as being both part of the drug structure and part of the linker. Without wanting to be bound by any theory the chemical structures according to the present invention do not contain groups corresponding to R¹⁷- R¹⁷. For example, when R¹⁷ is CONHNHX this moiety is only present once at the joining point between the linker and the drug structure.

In some cases, L comprises an attachment group for attachment to A and a protease cleavable portion. In some cases, L comprises a valine-citrulline unit. In some cases, L comprises maleimidocaproyl-valine-citrulline-p-aminobenzylcarbamate. In some cases, the double bond of the maleimide is reacted with a thiol group of a cysteine residue of the antibody A to form a sulphur-carbon bond in order to effect linkage of the linker L to the antibody A.

In some cases, L is of the formula VIII: 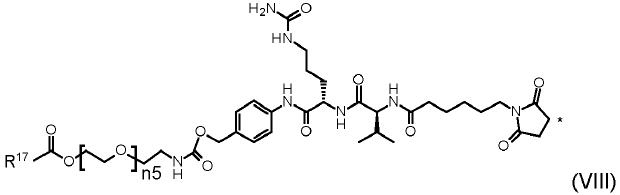
wherein * denotes the point of attachment to A;
R¹⁷ is directly or indirectly attached to drug D, preferably directly attached to drug D; and n5 is 1 to 9, preferably 3 to 5, more preferably 3 to 4.

In some cases, L is of the formula VII: 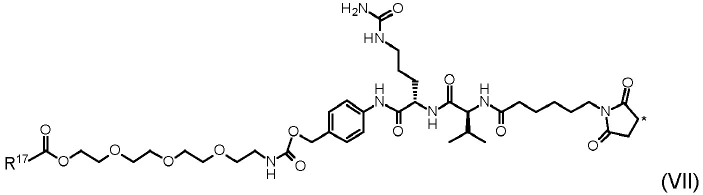 wherein * denotes the point of attachment to A and R¹⁷ is indirectly or directly attached to drug D. Preferably R¹⁷ is directly attached to drug D.

In some cases, -L-D has the following structure: 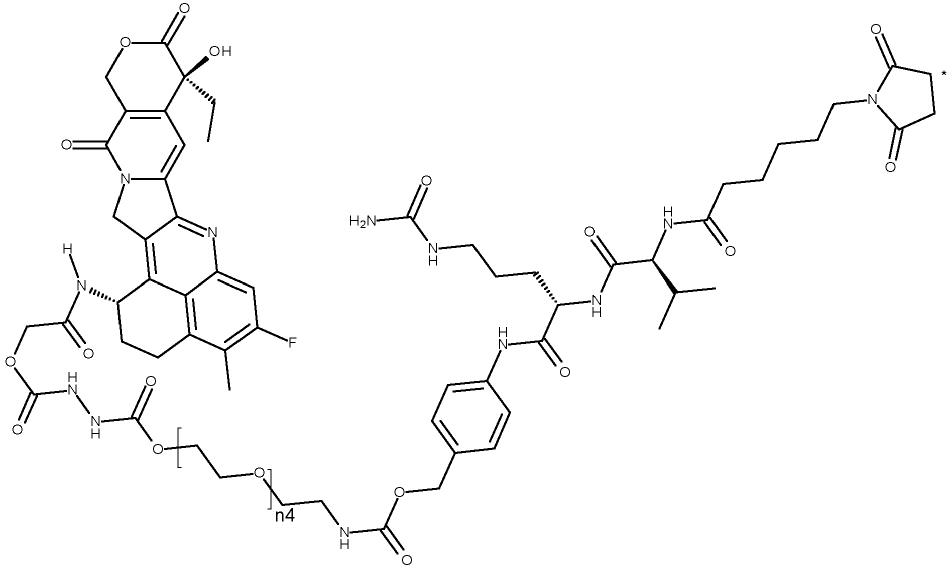 wherein * denotes the point of attachment to A and n4 is 1 to 9, preferably 3 to 5, more preferably 3 to 4.

Preferably, -L-D has the following structure:

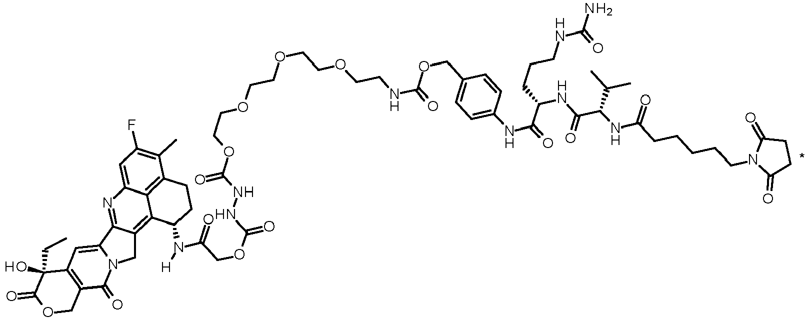

In certain cases, the conjugate is the conjugate described herein as OMTX-807.

In some cases, -L-D has the following structure: 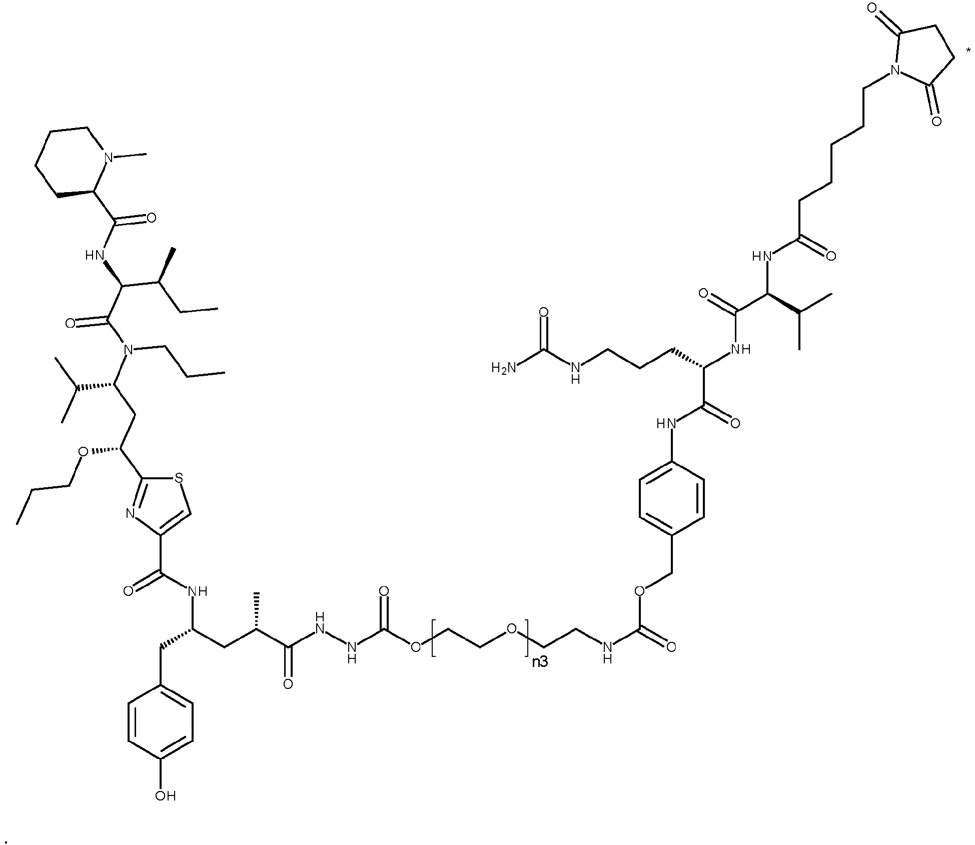 wherein * denotes the point of attachment to A and wherein n3 is 1 to 9, preferably 3 to 5, more preferably 3 to 4.

Preferably, -L-D has the following structure:

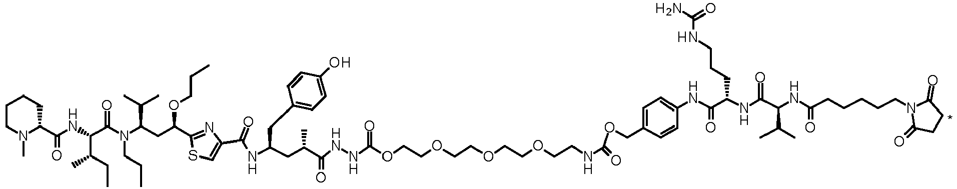

In certain cases, the conjugate is the conjugate described herein as OMTX707.

In some cases, wherein D is a topoisomerase inhibitor, L comprises a MC-GGFG linker. Preferably, the MC-GGFG linker is of structure: 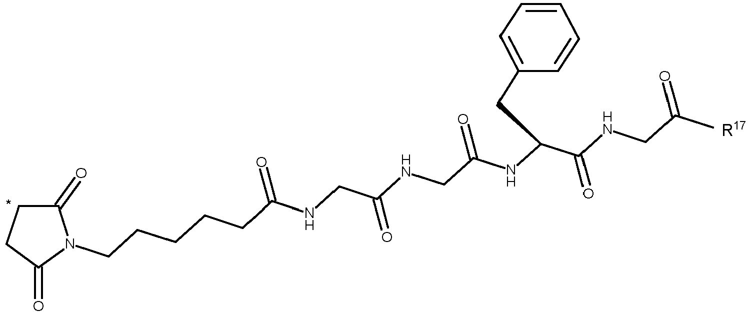
wherein * denotes the point of attachment to A;
R¹⁷ is directly or indirectly attached to drug D, preferably directly attached to drug D.

In a second aspect, the present invention provides a conjugate as defined in accordance with the first aspect of the invention for use in medicine.

In a third aspect, the present invention provides a conjugate as defined in accordance with the first aspect of the invention for use in a method of treatment of fibrosis or an inflammatory disease in a mammalian subject.

In some cases, the inflammatory disease is arthritis.

In a fourth aspect, the present invention provides a conjugate as defined in accordance with the first aspect of the invention for use in a method of treatment of a cancer, in a mammalian subject. In some cases, said conjugate is for simultaneous, sequential or separate administration with one or more other antitumor drugs.

In some cases, the cancer is an Endo180 expressing cancer. In some cases, the cancer is a solid tumor cancer.

In some cases, the cancer is a carcinoma. In some cases, the conjugate for use in a method of treating a carcinoma comprises a topoisomerase inhibitor, preferably a topoisomerase inhibitor according to the first aspect of the present invention. In some cases, the carcinoma is a non-small-cell lung cancer.

In some cases, the cancer is a sarcoma. In some cases, the conjugate for use in a method of treating a sarcoma comprises a cytolysin preferably a cytolysin according to the first aspect of the present invention. In some cases, the sarcoma is a soft-tissue sarcoma. In certain cases, the soft-tissue sarcoma is a leiomyosarcoma.

In a fifth aspect, the present invention provides a method of treating a cancer in a mammalian subject, comprising administering a therapeutically effective amount of a conjugate as defined in the first aspect to the subject in need thereof.

In a sixth aspect, the present invention provides the use of a conjugate as defined in accordance with the first aspect of the invention for use in the preparation of a medicament for treating a cancer in a mammalian subject, said treating comprising administering the conjugate to the subject in need thereof.

In a seventh aspect, the present invention provides a process for the production of a conjugate as defined in the first aspect, comprising:
(a) linking the antibody that specifically binds domain III of Endo180 to the linker;
(b) linking the topoisomerase inhibitor or cytolysin to the linker; and
(c) optionally, purifying and/or isolating the conjugate, wherein steps (a) and (b) can be performed in any order.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Figure 1****. Biochemical characterization of the IgG G7V molecule. A)** SDS-PAGE analysis of the IgG G7V molecule under reducing (R) and non-reducing (NR) conditions; M: marker. **B)** Size-exclusion chromatography (SEC) of purified IgG G7V by HPLC. C) Dynamic light scattering (DLS) of the IgG G7V molecule. Dashed line represents the aggregation point of IgG G7V at 59 °C.
**Figure 2****. Binding of IgG G7V to immobilized human Endo180 and HT1080-WT cells. A)** Binding of IgG G7V to immobilized human Endo180-His molecule was analyzed via ELISA. **B)** Binding of IgG G7V to HT1080-WT cells was analyzed via flow cytometry.
**Figure 3****. Biochemical characterization of the IgG G7V molecule.** A) SDS-PAGE analysis of the IgG G7V Δab molecule under reducing (R) and non-reducing (NR) conditions (12 % PAA; 6 µg for reducing and 4 µg for non-reducing conditions; M: marker). B) Size-exclusion chromatography (SEC) of purified IgG G7V Δab by HPLC.
**Figure 4****. Binding of IgG G7V Δab to immobilized human Endo180 and HT1080-WT cells.** A) Binding of IgG G7V Δab to immobilized human Endo180-moFc molecules was analyzed via ELISA. B) Binding of IgG G7V Δab to HT1080-WT cells was analyzed via flow cytometry.
**Figure 5****. Binding of anti-Endo180 antibodies to immobilized different domains (DI to DIV) of human Endo180. A)** Summary of binding analysis of Endo180 antibodies to human Endo180-moFc domains DI to DIV by ELISA. **B)** ELISA binding analysis of Endo180 antibodies to different human Endo180-moFc domains containing DI to DIV. C) ELISA analysis of Endo180 antibodies binding to domain II of human Endo180-moFc domain II.
**Figure 6****. Binding of anti-Endo180 Fab molecules to immobilized domains I-IV of human Endo180.** Binding of Fab G7V, 2H9, 5F4 and 9B7 to human Endo180 in the presence of pre-bound G7V was analyzed via ELISA.
**Figure 7****. Competition assay analyzing the binding of anti-Endo180 Fab molecules in the presence of IgG G7V.** Binding of Fab and IgG G7V, 2H9, 5F4 and 9B7 to human Endo180-moFc domains I-IV was analyzed via ELISA.
**Figure 8****. OMTX007-G7V epitope mapping.** Alignment of the protein sequence of domain III of human Endo180 (from amino acid 231 to 370) and MRC1 (human mannose receptor). The different regions (region 1, 2, 3, 4, 5, 8 and 9) that have exposed amino acid residues on the surface of both molecules are highlighted in grey.
**Figure 9****. Flow cytometry analysis of pHrodo-labeled anti-Endo180 IgGs using different cell lines.** Relative MFI of U2OS, T98G, MDA-MB231 and SKOV-3 cell lines for internalized fluorescently tagged IgGs G7V, 2H9, 5F4 and 9B7 over time.
**Figure 10****. Flow cytometry analysis of pHrodo-labeled anti-Endo180 IgGs using different cell lines.** Relative MFI of U2OS, T98G, MDA-MB231 and SKOV-3 cell lines for internalized fluorescently tagged IgGs G7V, 2H9, 5F4 and 9B7 over time at both 37°C and 4°C.
**Figure 11****. Binding of anti-Endo180 antibodies (OMTX007-G7V, OMTX707 and OMTX807) to Endo180 versus parental HEK293 cells analysed through flow cytometry analysis.** Relative MFI of parental non-transfected HEK293 cells and HEK293-Endo180 cells cell lines for internalized fluorescently tagged OMTX007, OMTX707 and OMTX807.
**Figure 12****. Cell-death induction assay using OMTX007-G7V naked antibody, OMTX707 and OMTX807 on Endo180-expressing and parental HEK293 cells.** Cell viability (%) over protein concentration of OMTX007, OMTX707 and OMTX807.
**Figure 13****. Tumor growth changes in human IC9LC1111 lung tumor patient-derived xenograft mice.** Tumor volume (mm³) over time following administration of control, OMTX807 (10mg/kg), OMTX807 (30mg/kg) and docetaxel. Doses, administration route and schedule are indicated in the figure.
**Figure 14****. Relative body weight variations in human IC9LC11 lung tumor xenograft model.** Relative murine body weight over time following administration of control, OMTX807 (10mg/kg), OMTX807 (30mg/kg) and docetaxel. Doses, administration route and schedule are indicated in the figure.
**Figure 15****. Tumor growth evolution upon treatment with anti-Endo180 OMTX707 ADC in humanSA4033 leiomyosarcoma tumor patient-derived xenograft mice.** Tumor volume (mm³) over time following administration of control, OMTX707 (10mg/kg) and OMTX707 (30mg/kg). Doses, administration route and schedule are indicated in the figure.
**Figure 16****. Body weight changes upon treatment with anti-Endo180 OMTX707 ADC in human SA4033 leiomyosarcoma tumor patient-derived xenograft mice.** Relative murine body weight over time following administration of control, OMTX707 (10mg/kg) and OMTX707 (30mg/kg). Doses, administration route and schedule are indicated in the figure.

### Sequences

| **SEQ ID NO:** | **Description** | **Sequence** |
|---|---|---|
| 1 | G7V heavy chain | EVQLVESGGGLVKPGGSLRLSCAASGFTFSSYSMNWVRQAPGKG LEWVANIKPDGSERHSVDSVKGRFTISRDNSKNSLYLQMNSLRAE DTAVYYCARPGAGRLDYWGQGTLVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPELLGGPSVFLFPPKPKDTLMISRTPEVTCVVVDVSHEDP EVKFNWYVDGVEVHNAKTKPREEQYNSTYRVVSVLTVLHQDWLN GKEYKCKVSNKALPAPIEKTISKAKGQPREPQVYTLPPSREEMTKN QVSLTCLVKGFYPSDIAVEWESNGQPENNYKTTPPVLDSDGSFFLY SKLTVDKSRWQQGNVFSCSVMHEALHNHYTQKSLSLSPGK |
| 2 | G7V light chain | SSELTQDPAVSVALGQTVRITCQGDSLRSYYASWYQQKPGQAPVL VVYGKNNRPSGIPDRFSGSSSGNTASLTITGAQAEDEADYYCNSR DSSGNPWAFGGGTKVTVLGQPKAAPSVTLFPPSSEELQANKATLV CLISDFYPGAVTVAWKADSSPVKAGVETTTPSKQSNNKYAASSYLS LTPEQWKSHRSYSCQVTHEGSTVEKTVAPTECS |
| 3 | G7V, A11, A10, A04, H03, B03, E06, B02, D12, G10 V_{H} | EVQLVESGGGLVKPGGSLRLSCAASGFTFSSYSMNWVRQAPGKG LEWVANIKPDGSERHSVDSVKGRFTISRDNSKNSLYLQMNSLRAE DTAVYYCARPGAGRLDYWGQGTLVTVSS |
| 4 | C10 V_{H} | EVQLVESGGGLVKPGGSLRLSCAASGFTFPSYAMNWVRQAPGKG LEWVANIKPNGNERHSVESVKGRFTISRDNSKNSLYLQMNSLRAE DTAVYYCARPGAGRLDYWGQGTLVTVSS |
| 5 | G7V L V_{L} | SSELTQDPAVSVALGQTVRITCQGDSLRSYYASWYQQKPGQAPVL VVYGKNNRPSGIPDRFSGSSSGNTASLTITGAQAEDEADYYCNSR DSSGNPWAFGGGTKVTVL |
| 6 | A11 V_{L} | SSELTQDPAVSVALGQTVRITCQGDSLRSHYASWYQQKPGQAPVL VVYFRNKRPSGIPDRFSGSSSGNTASLTITGAQAEDEADYYCNSR GSSGNPWAFGGGTKVTVL |
| 7 | A10 V_{L} | SSELTQDPAVSVALGQTVRITCQGDSLRSNFASWYQQKPGQAPVL VVYVKNGRPSGIPDRFSGSSSGNTASLTITGAQAEDEADYYCNSR NSSTHPWAFGGGTKVTVL |
| 8 | A04 V_{L} | SSELTQDPAVSVALGQTVRITCQGDRLRRNYASWYQQKPGQAPVL VVHGRNKRPSGIPDRFSGSSSGNTASLTITGAQAEDEADYYCNSR DRSGNPWAFGGGTKVTVL |
| 9 | H03 V_{L} | SSELTQDPAVSVALGQTVRITCQGDSLRSFYASWYQQKPGQAPVL VVYGKNKRPSGIPDRFSGSSSGNTASLTITGAQAEDEADYYCNSLG SSGNPPAFGGGTKVTVL |
| 10 | B03 V_{L} | SSELTQDPAVSVALGQTVRITCQGDRLRRNYASWYQQKPGQAPVL VVYGRNNRPSGIPDRFSGSSSGNTASLTITGAQAEDEADYYCNSR DGSGTPWAFGGGTKVTVL |
| 11 | E06 V_{L} | SSELTQDPAVSVALGQTVRITCQGDSLRRSYASWYQQKPGQAPVL VVYFKNNRPSGIPDRFSGSSSGNTASLTITGAQAEDEADYYCNSR GTSRNPWAFGGGTKVTVL |
| 12 | B02 V_{L} | SSELTQDPAVSVALGQTVRITCQGDSLRGYYASWYQQKPGQAPVL VVYGQNKRPSGIPDRFSGSSSGNTASLTITGAQAEDEADYYCNSR SRSRIPWAFGGGTKVTVL |
| 13 | D12 V_{L} | SSELTQDPAVSVALGQTVRITCQGDSLRGYYASWYQQKPGQAPVL VVYGRNSRPSGIPDRFSGSSSGNTASLTITGAQAEDEADYYCNSR NRSGHPWAFGGGTKVTVL |
| 14 | G10 V_{L} | SSELTQDPAVSVALGQTVRITCQGDSLRSYYASWYQQKPGQAPVL VMYFKNNRPSGIPDRFSGSSSGNTASLTITGAQAEDEADYYCNSR GSSRNPWAFGGGTKVTVL |
| 15 | C10 V_{L} | SSELTQDPAVSVALGQTVRITCQGDSLRSYFASWYQQKPGQAPVL VVYFKNNRPSGIPDRFSGSSSGNTASLTITGAQAEDEADYYCNSPN SGVISWAFGGGTKVTVL |
| 16 | CDRH1 - generalised | GFTFX₁SYX₂MN |
| 17 | CDRH2 - generalised | NIKPX₃GX₄ERHSVX₅SVKG |
| 18 | CDRH3 - generalised | PGAGRLDY |
| 19 | CDRL1 - generalised | QGDX₆LRX₇X₈X₉AS |
| 20 | CDRL2 - generalised | X₁₀X₁₁NX₁₂RPS |
| 21 | CDRL3 - generalised | NSX₁₃X₁₄X₁₅X₁₆X₁₇X₁₈X₁₉X₂₀A |
| 22 | G7V CDRH1 | GFTFSSYSMN |
| 23 | G7V CDRH2 | NIKPDGSERHSVDSVKG |
| 24 | G7V CDRH3 | PGAGRLDY |
| 25 | C10 CDRH1 | GFTFPSYAMN |
| 26 | C10 CDRH2 | NIKPNGNERHSVESVKG |
| 27 | C10 CDRH3 | PGAGRLDY |
| 28 | G7V CDRL1 | QGDSLRSYYAS |
| 29 | G7V CDRL2 | GKNNRPS |
| 30 | G7V CDRL3 | NSRDSSGNPWA |
| 31 | A11 CDRL1 | QGDSLRSHYAS |
| 32 | A11 CDRL2 | FRNKRPS |
| 33 | A11 CDRL3 | NSRGSSGNPWA |
| 34 | A10 CDRL1 | QGDSLRSNFAS |
| 35 | A10 CDRL2 | VKNGRPS |
| 36 | A10 CDRL3 | NSRNSSTHPWA |
| 37 | A04 CDRL1 | QGDRLRRNYAS |
| 38 | A04 CDRL2 | GRNKRPS |
| 39 | A04 CDRL3 | NSRDRSGNPWA |
| 40 | H03 CDRL1 | QGDSLRSFYAS |
| 41 | H03 CDRL2 | GKNKRPS |
| 42 | H03 CDRL3 | NSLGSSGNPPA |
| 43 | B03 CDRL1 | QGDRLRRNYAS |
| 44 | B03 CDRL2 | GRNNRPS |
| 45 | B03 CDRL3 | NSRDGSGTPWA |
| 46 | E06 CDRL1 | QGDSLRRSYAS |
| 47 | E06 CDRL2 | FKNNRPS |
| 48 | E06 CDRL3 | NSRGTSRNPWA |
| 49 | B02 CDRL1 | QGDSLRGYYAS |
| 50 | B02 CDRL2 | GQNKRPS |
| 51 | B02 CDRL3 | NSRSRSRIPWA |
| 52 | D12 CDRL1 | QGDSLRGYYAS |
| 53 | D12 CDRL2 | GRNSRPS |
| 54 | D12 CDRL3 | NSRNRSGHPWA |
| 55 | G10 CDRL1 | QGDSLRSYYAS |
| 56 | G10 CDRL2 | FKNNRPS |
| 57 | G10 CDRL3 | NSRGSSRNPWA |
| 58 | C10 CDRL1 | QGDSLRSYFAS |
| 59 | C10 CDRL2 | FKNNRPS |
| 60 | C10 CDRL3 | NSPNSGVISWA |
| 61 | Human Endo180 | MGPGRPAPAPWPRHLLRCVLLLGCLHLGRPGAPGDAALPEPNVFL IFSHGLQGCLEAQGGQVRVTPACNTSLPAQRWKWVSRNRLFNLG TMQCLGTGWPGTNTTASLGMYECDREALNLRWHCRTLGDQLSLL LGARTSNISKPGTLERGDQTRSGQWRIYGSEEDLCALPYHEVYTIQ GNSHGKPCTIPFKYDNQWFHGCTSTGREDGHLWCATTQDYGKDE RWGFCPIKSNDCETFWDKDQLTDSCYQFNFQSTLSWREAWASCE QQGADLLSITEIHEQTYINGLLTGYSSTLWIGLNDLDTSGGWQWSD NSPLKYLNWESDQPDNPSEENCGVIRTESSGGWQNRDCSIALPYV CKKKPNATAEPTPPDRWANVKVECEPSWQPFQGHCYRLQAEKRS WQESKKACLRGGGDLVSIHSMAELEFITKQIKQEVEELWIGLNDLKL QMNFEWSDGSLVSFTHWHPFEPNNFRDSLEDCVTIWGPEGRWN DSPCNQSLPSICKKAGQLSQGAAEEDHGCRKGWTWHSPSCYWL GEDQVTYSEARRLCTDHGSQLVTITNRFEQAFVSSLIYNWEGEYF WTALQDLNSTGSFFWLSGDEVMYTHWNRDQPGYSRGGCVALAT GSAMGLWEVKNCTSFRARYICRQSLGTPVTPELPGPDPTPSLTGS CPQGWASDTKLRYCYKVFSSERLQDKKSWVQAQGACQELGAQLL SLASYEEEHFVANMLNKIFGESEPEIHEQHWFWIGLNRRDPRGGQ SWRWSDGVGFSYHNFDRSRHDDDDIRGCAVLDLASLQWVAMQC DTQLDWICKIPRGTDVREPDDSPQGRREWLRFQEAEYKFFEHHST WAQAQRICTWFQAELTSVHSQAELDFLSHNLQKFSRAQEQHWWI GLHTSESDGRFRWTDGSIINFISWAPGKPRPVGKDKKCVYMTASR EDWGDQRCLTALPYICKRSNVTKETQPPDLPTTALGGCPSDWIQF LNKCFQVQGQEPQSRVKWSEAQFSCEQQEAQLVTITNPLEQAFIT ASLPNVTFDLWIGLHASQRDFQWVEQEPLMYANWAPGEPSGPSP APSGNKPTSCAVVLHSPSAHFTGRWDDRSCTEETHGFICQKGTDP SLSPSPAALPPAPGTELSYLNGTFRLLQKPLRWHDALLLCESRNAS LAYVPDPYTQAFLTQAARGLRTPLWIGLAGEEGSRRYSWVSEEPL NYVGWQDGEPQQPGGCTYVDVDGAWRTTSCDTKLQGAVCGVSS GPPPPRRISYHGSCPQGLADSAWIPFREHCYSFHMELLLGHKEAR QRCQRAGGAVLSILDEMENVFVWEHLQSYEGQSRGAWLGMNFN PKGGTLVWQDNTAVNYSNWGPPGLGPSMLSHNSCYWIQSNSGL WRPGACTNITMGVVCKLPRAEQSSFSPSALPENPAALVVVLMAVLL LLALLTAALILYRRRQSIERGAFEGARYSRSSSSPTEATEKNILVSD MEMNEQQE |
| 62 | Murine Endo180 | MVPIRPALAPWPRHLLRCVLLLGGLRLGHPADSAAALLEPDVFLIFS QGMQGCLEAQGVQVRVTPVCNASLPAQRWKWVSRNRLFNLGAT QCLGTGWPVTNTTVSLGMYECDREALSLRWQCRTLGDQLSLLLG ARASNASKPGTLERGDQTRSGHWNIYGSEEDLCARPYYEVYTIQG NSHGKPCTIPFKYDNQWFHGCTSTGREDGHLWCATTQDYGKDER WGFCPIKSNDCETFWDKDQLTDSCYQFNFQSTLSWREAWASCEQ QGADLLSITEIHEQTYINGLLTGYSSTLWIGLNDLDTSGGWQWSDN SPLKYLNWESDQPDNPGEENCGVIRTESSGGWQNHDCSIALPYVC |
| | | KKKPNATVEPIQPDRWTNVKVECDPSWQPFQGHCYRLQAEKRSW QESKRACLRGGGDLLSIHSMAELEFITKQIKQEVEELWIGLNDLKLQ MNFEWSDGSLVSFTHWHPFEPNNFRDSLEDCVTIWGPEGRWNDS PCNQSLPSICKKAGRLSQGAAEEDHGCRKGWTWHSPSCYWLGE DQVIYSDARRLCTDHGSQLVTITNRFEQAFVSSLIYNWEGEYFWTA LQDLNSTGSFRWLSGDEVIYTHWNRDQPGYRRGGCVALATGSAM GLWEVKNCTSFRARYICRQSLGTPVTPELPGPDPTPSLTGSCPQG WVSDPKLRHCYKVFSSERLQEKKSWIQALGVCRELGAQLLSLASY EEEHFVAHMLNKIFGESEPESHEQHWFWIGLNRRDPREGHSWRW SDGLGFSYHNFARSRHDDDDIRGCAVLDLASLQWVPMQCQTQLD WICKIPRGVDVREPDIGRQGRLEWVRFQEAEYKFFEHHSSWAQAQ RICTWFQADLTSVHSQAELDFLGQNLQKLSSDQEQHWWIGLHTLE SDGRFRWTDGSIINFISWAPGKPRPIGKDKKCVYMTARQEDWGDQ RCHTALPYICKRSNSSGETQPQDLPPSALGGCPSGWNQFLNKCFR IQGQDPQDRVKWSEAQFSCEQQEAQLVTIANPLEQAFITASLPNVT FDLWIGLHASQRDFQWIEQEPLLYTNWAPGEPSGPSPAPSGTKPT SCAVILHSPSAHFTGRWDDRSCTEETHGFICQKGTDPSLSPSPAAT PPAPGAELSYLNHTFRLLQKPLRWKDALLLCESRNASLAHVPDPYT QAFLTQAARGLQTPLWIGLASEEGSRRYSWLSEEPLNYVSWQDEE PQHSGGCAYVDVDGTWRTTSCDTKLQGAVCGVSRGPPPRRINYR GSCPQGLADSSWIPFREHCYSFHMEVLLGHKEALQRCQKAGGTVL SILDEMENVFVWEHLQTAEAQSRGAWLGMNFNPKGGTLVWQDNT AVNYSNWGPPGLGPSMLSHNSCYWIQSSSGLWRPGACTNITMGV VCKLPRVEENSFLPSAALPESPVALVVVLTAVLLLLALMTAALILYRR RQSAERGSFEGARYSRSSHSGPAEATEKNILVSDMEMNEQQE |
| 63 | Human Endo180 epitope | SDQPDNPSE |
| 64 | Human Endo180 (alignment fragment from Fig. 8) | KSNDCETFWDKDQLTDSCYQFNFQSTLSWREAWASCEQQGADLL SITEIHEQTYINGLLTGYSSTLWIGLNDLDTSGGWQWSDNSPLKYL NWESDQPDNPSEENCGVIRTESSGGWQNRDCSIALPYVCKKKPN ATAEPT |
| 65 | Human MRC1 receptor (alignment fragment from Fig. 8) | KFEGSESLWNKDPLTSVSYQINSKSALTWHQARKSCQQQNAELLS ITEIHEQTYLTGLTSSLTSGLWIGLNSLSFNSGWQWSDRSPFRYLN WLPGSPSAEPGKSCVSLNPGKNAKWENLECVQKLGYICKKGNTTL NSFV |

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

### DEFINITIONS

Examples of substituents are described in more detail below.

Unless otherwise stated, halo is selected from chloro (Cl), fluoro (F), bromo (Br) and iodo (I), such as fluoro or chloro.

Hydroxy: -OH.

Phenyl: an aromatic functional group derived from benzene (C₆).

Unless otherwise specified, the term "substituted" as used herein, pertains to a parent group which bears one or more substituents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known.

The term "alkyl" refers to a saturated linear or branched hydrocarbon group, preferably comprising 1 to 20 carbon atoms. The term "alkyl" is used to refer both to monovalent hydrocarbon groups (commonly known as "alkyl" groups) and to divalent hydrocarbon groups (commonly known as "alkylene" groups). The terms "alkyl" and "alkylene" are used interchangeably herein.

The term "C₁₋₆ alkyl" as used herein, pertains to a monovalent or bivalent moiety obtained by removing one or more hydrogen atoms from a carbon atom of a hydrocarbon compound having from 1 to 6 carbon atoms, which are saturated and may also be branched. The term "C₁₋₄ alkyl" as used herein, pertains to a monovalent or bivalent moiety obtained by removing one or more hydrogen atoms from a carbon atom of a hydrocarbon compound having from 1 to 4 carbon atoms, which are saturated.

Examples of saturated alkyl groups include, but are not limited to, methyl (C₁), ethyl (C₂), propyl (C₃), butyl (C₄), pentyl (C₅) and hexyl (C₆).

Alkyl groups may be linear or branched.

Examples of saturated linear alkyl groups include, but are not limited to, methyl (C₁), ethyl (C₂), n-propyl (C₃), n-butyl (C₄), n-pentyl (amyl) (C₅) and n-hexyl (C₆).

Examples of saturated branched alkyl groups include iso-propyl (C₃), iso-butyl (C₄), sec-butyl (C₄), tert-butyl (C₄), iso-pentyl (C₅), and neo-pentyl (C₅).

Alkyl groups may be substituted or unsubstituted. Within the meaning of this invention unless explicitly defined in the formulae the alkyl groups are preferably unsubstituted.

C₁₋₄ alkoxy: The term C₁₋₄ alkoxy as used herein, pertains to an OR group, wherein R is an C₁₋₄ hydrocarbon group. Examples of C₁₋₄ alkoxy groups include, but are not limited to, OMe, OEt (ethoxy), - O(nPr) (n-propoxy), -O(iPr) (isopropoxy), O(nBu) (n-butoxy).
Oxyalkylene: O-alkylene

Fused ring: refers to a C₃₋₁₂ cycloalkyl or C₃₋₁₀ heterocyclyl; preferably a C₃₋₈ cycloalkyl or C₃₋₈ heterocyclyl which is attached to another ring system.

The term "C₃₋₁₂ cycloalkyl" as used herein, pertains to an alkyl group which is also a cyclyl group; that is, a cyclic hydrocarbon (carbocyclic) compound, which moiety has from 3 to 7 carbon atoms, including from 3 to 7 ring atoms. The carbocyclic ring may be saturated or unsaturated.

Examples of cycloalkyl groups include, but are not limited to, those derived from:
saturated monocyclic hydrocarbon compounds:
   cyclopropane (C₃), cyclobutane (C₄), cyclopentane (C₅), cyclohexane (C₆), cycloheptane (C₇),
   methylcyclopropane (C₄), dimethylcyclopropane (C₅), methylcyclobutane (C₅), dimethylcyclobutane (C₆),
   methylcyclopentane (C₆), dimethylcyclopentane (C₇) and methylcyclohexane (C₇);
unsaturated monocyclic hydrocarbon compounds:
   cyclopropene (C₃), cyclobutene (C₄), cyclopentene (C₅), cyclohexene (C₆), methylcyclopropene (C₄),
   dimethylcyclopropene (C₅), methylcyclobutene (C₅), dimethylcyclobutene (C₆), methylcyclopentene (C₆),
   dimethylcyclopentene (C₇) and methylcyclohexene (C₇); and
saturated polycyclic hydrocarbon compounds:
   norcarane (C₇), norpinane (C₇), norbornane (C₇).

The term "C₃₋₁₀ heterocyclyl" as used herein, pertains to a heterocyclic compound, which has from 3 to 10 ring atoms, of which from 1 to 5 are ring heteroatoms. In certain embodiments, each ring has from 3 to 7 ring atoms, of which from 1 to 4 are ring heteroatoms. The ring may be saturated or unsaturated, and may be bridged or unbridged. The ring may be a fused ring or a single ring. For the avoidance of doubt, substituents on the heterocycloalkyl ring may be linked via either a carbon atom or a heteroatom.

In this context the term 'heteroatom' means O, S, N, Si or B (Boron).

In this context, the prefixes (e.g. C₃₋₁₀ C₃₋₇, C₅₋₆, etc.) denote the number of ring atoms, or range of number of ring atoms, whether carbon atoms or heteroatoms. For example, the term "C₅₋₆ heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

Aryl groups may be a heteroaryl group.

In this context, the prefixes (e.g. C₃₋₁₀ C₃₋₇, C₅₋₆, etc.) denote the number of ring atoms, or range of number of ring atoms, whether carbon atoms or heteroatoms. For example, the term "C₅₋₆ heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

The term "aryl," as used herein, represents a monovalent monocyclic, bicyclic, or multicyclic ring system formed by carbon atoms, wherein the ring attached to the pendant group is aromatic. Examples of aryl groups are phenyl, naphthyl, phenanthrenyl, and anthracenyl. An aryl ring can be attached to its pendant group at any heteroatom or carbon ring atom that results in a stable structure and any of the ring atoms can be optionally substituted unless otherwise specified.

Aryl groups may be substituted or unsubstituted. Without wanting to be bound by any theory, within the meaning of this invention, the aryl groups are preferable unsubstituted.

The terms n1-n6 are used to refer to the number of repeating structural units, for example repeating -(OCH₂CH₂)- or -(CH₂)- units.

### Endo180

As used herein, "Endo180", also known as uPARAP, may be an Endo180 of any mammalian species. In some cases, Endo180 is human Endo180, the amino acid sequence of which is disclosed at UniProt accession No. Q9UBG0 (Version 172, dated 8 November 2023) and in SEQ ID NO: 61. A molecule that binds Endo180 (e.g., an antibody conjugate described herein) may bind to domain III of Endo180. Domain III of Endo180 corresponds to the first C-type lectin-like domain. In some cases, Endo180 is murine Endo180, the amino acid sequence of which is disclosed at UniProt accession No. Q64449 (Version 171, dated 8 November 2023) and in SEQ ID NO: 62.

### Conjugate

As used herein "conjugate" includes the resultant structure formed by linking molecules and specifically includes antibody-drug conjugates (ADCs).

### Specifically and selectively binds

The term "specifically binds" refers to the binding of an antibody, or a binding fragment thereof, that binds a particular epitope without substantially binding to another epitope. For example, the antibody, or binding fragment thereof that binds to Endo180, e.g. domain III of Endo180, has a dissociation constant (K_{D}) of less than 500nM.

The terms "selectively binds" and "selective binding" refer to binding of an antibody, or binding fragment thereof, to a predetermined molecule (e.g. an antigen) in a specific manner. For example, the antibody, or binding fragment thereof, may bind to Endo180, e.g. domain III of Endo180, with an affinity represented by a K_{D} of less than 500nM, and may bind to the predetermined molecule with an affinity that is at least two-fold greater (e.g. five-fold or ten-fold greater) than its affinity for binding to a molecule other than the predetermined molecule.

### Antibody molecule

As used herein with reference to all aspects of the invention, the term "antibody" or "antibody molecule" includes any immunoglobulin whether natural or partly or wholly synthetically produced. The term "antibody" or "antibody molecule" includes monoclonal antibodies (mAb) and polyclonal antibodies (including polyclonal antisera). Antibodies may be intact, or fragments derived from full antibodies (see below). Antibodies may be human antibodies, humanised antibodies, or antibodies of non-human origin. "Monoclonal antibodies" are homogeneous, highly specific antibody populations directed against a single antigenic site or "determinant" of the target molecule. "Polyclonal antibodies" include heterogeneous antibody populations that are directed against different antigenic determinants of the target molecule. The term "antiserum" or "antisera" refers to blood serum containing antibodies obtained from immunized animals.

It has been shown that fragments of a whole antibody can perform the function of binding antigens. Thus reference to antibody herein, and with reference to the methods, arrays and kits of the invention, covers a full antibody and also covers any polypeptide or protein comprising an antibody binding fragment. Examples of binding fragments are (i) the Fab fragment consisting of V_{L}, V_{H}, C_{L} and C_{H}1 domains; (ii) the Fd fragment consisting of the V_{H} and C_{H}1 domains; (iii) the fragment consisting of the V_{L} and C_{L} domains (the light chain) (iv) the Fv fragment consisting of the V_{L} and V_{H} domains of a single antibody; (v) the dAb fragment which consists of a V_{H} domain; (vi) isolated CDR regions; (vii) F(ab')₂ fragments, a bivalent fragment comprising two linked Fab fragments (viii) single chain Fv molecules (scFv), wherein a V_{H} domain and a V_{L} domain are linked by a peptide linker which allows the two domains to associate to form an antigen binding site; (ix) bispecific single chain Fv dimers (WO 93/11161) and (x) "diabodies", multivalent or multispecific fragments constructed by gene fusion (WO94/13804; 58). Fv, scFv or diabody molecules may be stabilised by the incorporation of disulphide bridges linking the VH and VL domains. Minibodies comprising a scFv joined to a CH3 domain may also be made.

### Affinity

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (K_{D}). Affinity can be measured by common methods known in the art, including those described herein. Specific illustrative and exemplary embodiments for measuring binding affinity are known in the art, any of which can be used for purposes of the present invention.

One illustrative method for measuring reactivity is where a "K_{D}" or "K_{D} value" may be measured by the Octet method.

To determine assay parameters using antibodies and human Endo I-IV antigen the assay will be set up by titrating of human Endo I-IV antigen, immobilization to sensor (loading), association in soluble Fab antibody (One concentration), dissociation in assay blocking buffer and finally determination of optimal antigen loading to sensor.

For the affinity measurement, the antigen is immobilized to sensor, based on assay setup, antibodies titrated (7pt titration), association measured in soluble Fab antibody, dissociation in assay blocking buffer and calculation of Kon, Koff, KD choosing appropriate concentrations for affinity calculation.

### Cytotoxic chemotherapeutic agents

In some cases, in accordance with any aspect of the present invention, the conjugate of the invention may be administered with, or for administration with, (whether simultaneously, sequentially or separately) other antitumor drugs, including, but not limited to, a cytotoxic chemotherapeutic agent or an anti-angiogenic agent or an immunotherapeutic agent.

Cytotoxic chemotherapeutic agents are well known in the art and include anti-cancer agents such as:
Alkylating agents including nitrogen mustards such as mechlorethamine (HN2), cyclophosphamide, ifosfamide, melphalan (L-sarcolysin) and chlorambucil; 10 ethylenimines and methylmelamines such as hexamethylmelamine, thiotepa; alkyl sulphonates such as busulfan; nitrosoureas such as carmustine (BCNU), lomustine (CCNLJ), semustine (methyl-CCN-U) and streptozoein (streptozotocin); and triazenes such as 26acarbazine (DTIC; dimethyltriazenoimidazolecarboxamide);
Antimetabolites including folic acid analogues such as methotrexate (amethopterin); pyrimidine analogues such as fluorouracil (5- fluorouracil; 5-FU), floxuridine (fluorodeoxyuridine; FudR) and cytarabine (cytosine arabinoside); and purine analogues and related inhibitors such as mercaptopurine (6-mercaptopurine; 6-MP), thioguanine (6-thioguanine; TG) and pentostatin (2'-deoxycofonnycin). Natural Products including vinca alkaloids such as vinblastine (VLB) and vincristine; epipodophyllotoxins such as etoposide and teniposide; antibiotics such as dactinomycin (actinomycin D), daunorabicin (daunomycin; rubidomycin), doxorubicin, bleomycin, plicamycin (mithramycin) and mitomycin (mitomycin Q; enzymes such as L-asparaginase; and biological response modifiers such as interferon alphenomes. Miscellaneous agents including platinum coordination complexes such as cisplatin (cis-DDP) and carboplatin; anthracenedione such as mitoxantrone and anthracycline; substituted urea such as hydroxyurea; methyl hydrazine derivative such as procarbazine (N- methylhydrazine, MIH); and adrenocortical suppressant such as mitotane (o, p'-DDD) and aminoglutethimide; taxol and analogues/derivatives; and hormone agonists/antagonists such as flutamide and tamoxifen. A further preferred cytotoxic agent is Gemcitabine (Gemzar^{®}). A further preferred cytotoxic agent is Paclitaxel bound to human serum albumin (Abraxane^{®}).

Anti-angiogenic agents are well known in the art and include anti-cancer agents such as bevacizumab, itraconazole, and carboxyamidotriazole.

Immunotherapeutic agents are known in the art and include, for example, anti-programmed cell death protein 1 (PD-1) antibodies and anti-programmed death-ligand 1 (PD-L1) antibodies, including Nivolumab (MDX1106) and Pembrolizumab (MK-3475).

### Pharmaceutical compositions

The conjugates of the present invention may be comprised in pharmaceutical compositions with a pharmaceutically acceptable excipient.

A pharmaceutically acceptable excipient may be a compound or a combination of compounds entering into a pharmaceutical composition which does not provoke secondary reactions, and which allows, for example, facilitation of the administration of the conjugate, an increase in its lifespan and/or in its efficacy in the body or an increase in its solubility in solution. These pharmaceutically acceptable vehicles are well known and will be adapted by the person skilled in the art as a function of the mode of administration of the conjugate.

In some embodiments, conjugates of the present invention may be provided in a lyophilised form for reconstitution prior to administration. For example, lyophilised conjugates may be re-constituted in sterile water and mixed with saline prior to administration to an individual.

Conjugates of the present invention will usually be administered in the form of a pharmaceutical composition, which may comprise at least one component in addition to the conjugate. Thus pharmaceutical compositions may comprise, in addition to the conjugate, a pharmaceutically acceptable excipient, carrier, buffer, stabilizer or other materials well known to those skilled in the art. Such materials should be non-toxic and should not interfere with the efficacy of the conjugate. The precise nature of the carrier or other material will depend on the route of administration, which may be by bolus, infusion, injection, or any other suitable route, as discussed below.

For intra-venous administration, e.g. by injection, the pharmaceutical composition comprising the conjugate may be in the form of a parenterally acceptable aqueous solution which is pyrogen-free and has suitable pH, isotonicity and stability. Those of relevant skill in the art are well able to prepare suitable solutions using, for example, isotonic vehicles, such as Sodium Chloride Injection, Ringer's Injection, Lactated Ringer's Injection. Preservatives, stabilizers, buffers, antioxidants and/or other additives may be employed as required including buffers such as phosphate, citrate and other organic acids; antioxidants, such as ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3'-pentanol; and m-cresol); low molecular weight polypeptides; proteins, such as serum albumin, gelatin or immunoglobulins; hydrophilic polymers, such as polyvinylpyrrolidone; amino acids, such as glycine, glutamine, asparagine, histidine, arginine, or lysine; monosaccharides, disaccharides and other carbohydrates including glucose, mannose or dextrins; chelating agents, such as EDTA; sugars, such as sucrose, mannitol, trehalose or sorbitol; salt-forming counter-ions, such as sodium; metal complexes (e.g. Zn-protein complexes); and/or non-ionic surfactants, such as TWEEN^{™}, PLURONICS^{™} or polyethylene glycol (PEG).

### Subject

The subject may be a human, a companion animal (e.g. a dog or cat), a laboratory animal (e.g. a mouse, rat, rabbit, pig or non-human primate), a domestic or farm animal (e.g. a pig, cow, horse or sheep). Preferably, the subject is a human. In some cases, the subject may be a human diagnosed with or classified as being at risk of developing a cancer, e.g., an epithelial tumor, a solid tumor or a blood neoplasm. In certain cases, the subject may be a laboratory animal, e.g., a mouse model of a cancer.

### Cancer

In some cases, in accordance with the present invention, the anti-Endo180 conjugates described herein may be for use in the treatment of a cancer in a mammalian subject. The cancer may be an Endo180 expressing cancer. The cancer may be, for example, a solid tumor cancer. In certain cases, the cancer may be a carcinoma such as a non-small-cell lung cancer. In certain cases, the cancer may be a sarcoma, such as a soft-tissue sarcoma and particularly a leiomyosarcoma.

### Fibrosis

In some cases, in accordance with the present invention, the anti-Endo180 conjugates described herein may be for use in the treatment of fibrosis. Without wishing to be bound by theory, Endo180 is likely to contribute to the accumulation of collagen and other ECM components in fibrosis, leading to organ damage.

The present inventors believe that the anti-Endo180 antibodies described herein, and/or conjugates thereof described herein, are able to ameliorate fibrosis.

### Inflammatory condition

In some cases, in accordance with the present invention, the anti-Endo180 conjugates described herein may be for use in the treatment of an inflammatory disease. Endo180 has been described as promoting the recruitment and activation of inflammatory cells in inflammatory diseases. The inflammatory disease may be one wherein Endo180 is overexpressed. In certain cases, the inflammatory disease is arthritis.

The present inventors believe that the anti-Endo180 antibodies described herein, and/or conjugates thereof described herein, are able to ameliorate inflammatory diseases such as arthritis.

### Examples

### EXAMPLE 1: Generation and production of humanized anti-Endo180 antibody

Human IgG1 G7V with a wildtype γ1 heavy chain was transiently produced in HEK293-6E suspension cells. Sequence of heavy and light chain are shown below.
Heavy chain: 
EVQLVESGGGLVKPGGSLRLSCAASGFTFSSYSMNWVRQAPGKGLEWVANIKPDGSERHSVDSVKGR
FTISRDNSKNSLYLQMNSLRAEDTAVYYCARPGAGRLDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPPVLDSDGSFFLYSKLTVDK
SRWQQGNVFSCSVMHEALHNHYTQKSLSLSPGK
Light chain:

SSELTQDPAVSVALGQTVRITCQGDSLRSYYASWYQQKPGQAPVLVVYGKNNRPSGIPDRFSGSSSGN
TASLTITGAQAEDEADYYCNSRDSSGNPWAFGGGTKVTVLGQPKAAPSVTLFPPSSEELQANKATLVCLI
SDFYPGAVTVAWKADSSPVKAGVETTTPSKQSNNKYAASSYLSLTPEQWKSHRSYSCQVTHEGSTVEK
TVAPTECS

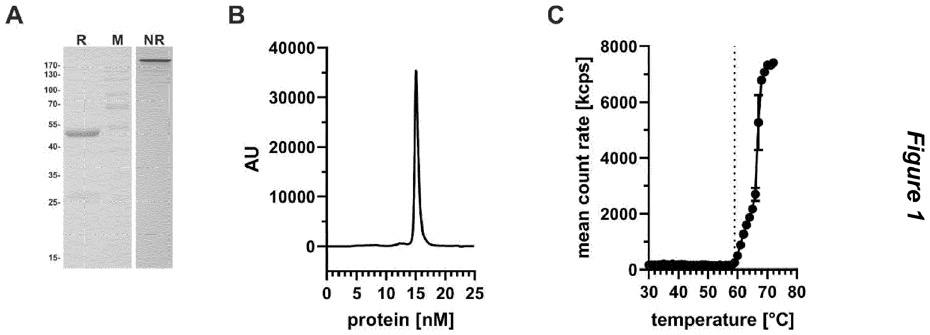

Protein was purified from a lab scale culture via protein A affinity chromatography, eluted with acidic buffer and dialyzed against PBS. The purified IgG was analysed through SDS-PAGE, and two bands at approximately 50 kDa and 27 kDa were detected under reducing conditions, representing the heavy (calculated weight: 48.8 kDa) and the light (calculated weight: 22.7 kDa) chains of the antibody (Fig. 1A). Under non-reducing conditions, one band at approximately 200 kDa was detected, representing the complete IgG molecule. Purified IgG G7V was further analyzed via analytical size-exclusion chromatography by HPLC (Fig. 1B), showing a major peak corresponding to the intact IgG G7V molecule. Finally, thermal stability of the IgG G7V molecule was analyzed in dynamic light scattering (DLS) (Fig. 1C).

The binding of IgG G7V was analyzed via ELISA using in-house produced human Endo180-His (aa: 31-513; comprising the first four N-terminal domains) as immobilized antigen. The IgG G7V antibody bound to human Endo180 in a concentration-dependent manner with an EC₅₀ value of 4.7 nM.

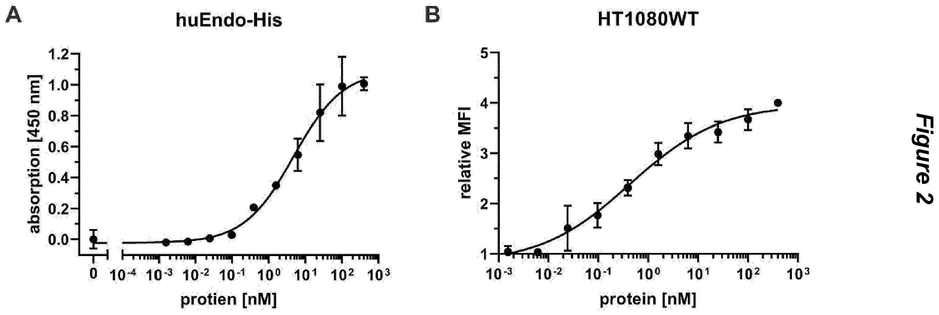

The binding of the IgG G7V to Endo180-expressing HT1080-WT cells was further analyzed by flow cytometry. IgG G7V antibody bound to HT1080-WT cells in a concentration-dependent manner with an EC₅₀ value of 0.4 nM (Figure 2). The results of IgG G7V analysis is shown below in Table 1.

**Table 1: Biochemical and bioactivity of IgG G7V antibody. Mean; n=1.**

| **Production vol. [ml]** | **conc. [mg/ml]** | **amount [mg]** | **yield [mg/l]** | **Aggregation point [°C]** | **ELISA (EC₅₀)** | **FACS** (EC₅₀) |
|---|---|---|---|---|---|---|
| | | | | | **huEndo** | **HT1080-WT** |
| 100 ml | 0.8 | 1.0 | 10.0 | 59 | 4.7 nM | 0.4 nM |

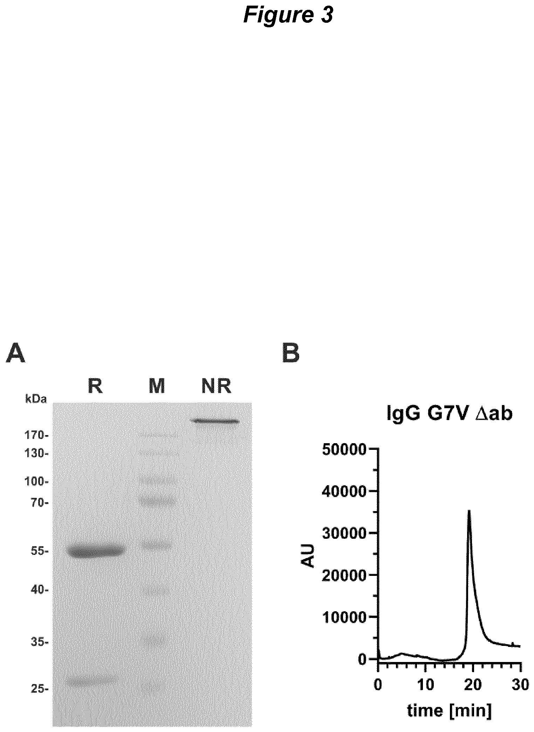

To produce an IgG with a silent Fc (based on Δab mutations, Armour et al., 1999) the two plasmids encoding IgG G7V Δab heavy and light chain were co-transfected into HEK293-6E cells and IgG was purified by protein A affinity chromatography. Purified IgG G7V Δab was analyzed via SDS-PAGE revealing two bands at approximately 50 kDa and 27 kDa under reducing conditions, corresponding to the heavy and light chain of the antibody (Fig. 3A). Under non-reducing conditions, one band at approximately 200 kDa was detected corresponding to the complete antibody. The purified antibody was also analyzed via analytical size-exclusion chromatography by HPLC (Fig. 3B), revealing a major peak corresponding to the intact IgG G7V molecule.

The binding of IgG G7V Δab was analyzed via ELISA using In-house produced human Endo180-mouse Fc (aa: 31-513; comprising the first four N-terminal domains, aa: 31-370; comprising the first three domains, aa: 31-232; comprising the first two domains, aa: 31-173; comprising the first domains) as immobilized antigen. The IgG G7V Δab antibody bound to immobilized antigens (domain I-IV and domain I-III) in a concentration-dependent manner with EC₅₀ values of 0.53 nM and 0.45 nM respectively (Table 2).

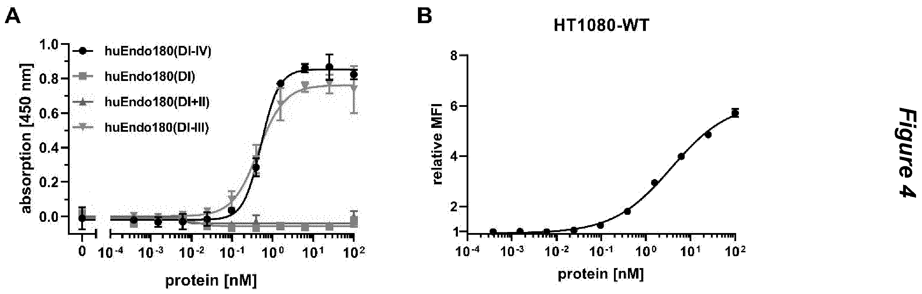

The binding of the IgG G7V Δab to HT1080-WT cells was further analyzed by flow cytometry. IgG G7V Δab antibody bound to HT1080-WT cells in a concentration-dependent manner with EC₅₀ values of 3.7 nM (Figure 4). The results of this analysis are shown below in Table 2.

**Table 2: Biochemical and bioactivity of IgG G7V Δab antibody. Mean; n=1.**

| **IgG G7V** | **conc. [mg/ml]** | **amount [mg]** | **ELISA (EC₅₀)** | **ELISA (EC₅₀)** | **FACS (EC₅₀)** |
|---|---|---|---|---|---|
| | | | **huEndo180 (DI-IV)** | **huEndo180 (DI-III)** | **HT1080-WT** |
| | 4.0 | 11.2 | 0.53 nM | 0.45 nM | 3.7 nM |

For larger scale production (1L), DNA fragment corresponding to heavy and light chains of IgG G7V were introduced into plasmids pEG3.6-EC2203A-HC-LC, pEG11.6-EC2203A-HC-LC and pEG11.6-EC2203A-OMTX-HC-LC for stable expression and production into Eirgenix's CHO expression system (EG CHO K1 sv).

After stable single clone generation, screening and selection, the selected clones have a productivity of around 2.5-3 mg/mL of IgG G7V antibody protein.

The antibody was manufactured at 1L scale, and the protein purified from 850mL cell culture. Purification from clarified supernatant of cell culture was done by Protein A affinity chromatography using MabSelect SuRe LX column. After purification, the purified antibody was concentrated at 10.59 mg/mL and the purity was analyzed by SEC-HPLC. The monomer content was 98.8% and aggregates 1.2%. The endotoxin levels obtained were 2.61 EU/mg of protein.

### EXAMPLE 2: Binding Domain and epitope mapping and Internalization

G7V antibody was compared to other anti-Endo180 antibodies (IgG 2H9, IgG 5F4 and IgG 9B7) for binding to different human Endo180 molecules in ELISA experiments. In this experiment, human Endo180 molecules, fused to murine Fc domain, containing domains I-IV (aa 31-513), domains I-III (aa 31-370), domains I-II (aa 31-232) and domain I (aa 31-173) were used as immobilized antigens and the different anti-Endo180 antibodies were titrated from 0.1 pM to 100 nM.

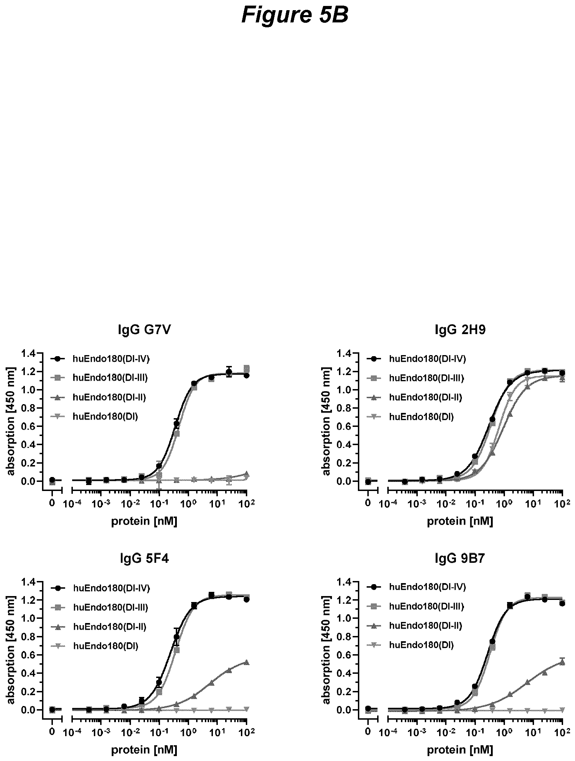

ELISA-binding experiments of IgG G7V showed binding to human Endo180-moFc domains I-IV and I-III and no binding to domains I-II and domains I or II, indicating that the epitope for this antibody is located in domain III. IgG 2H9 showed binding to all domains containing domain I of human Endo180 indicating the epitope is located in domain I for this antibody. IgG 5F4 and IgG 9B7 showed binding to human Endo180-moFc domains I-IV and I-III and reduced binding to domains I-II and II. No binding was detected for domain I. Thus, the epitope for these two antibodies is located in domains II and III. (Figure 5B)

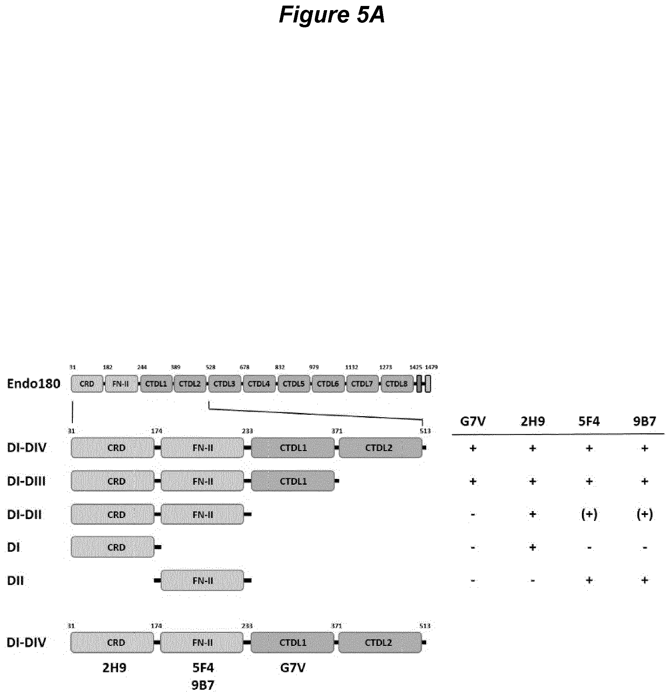

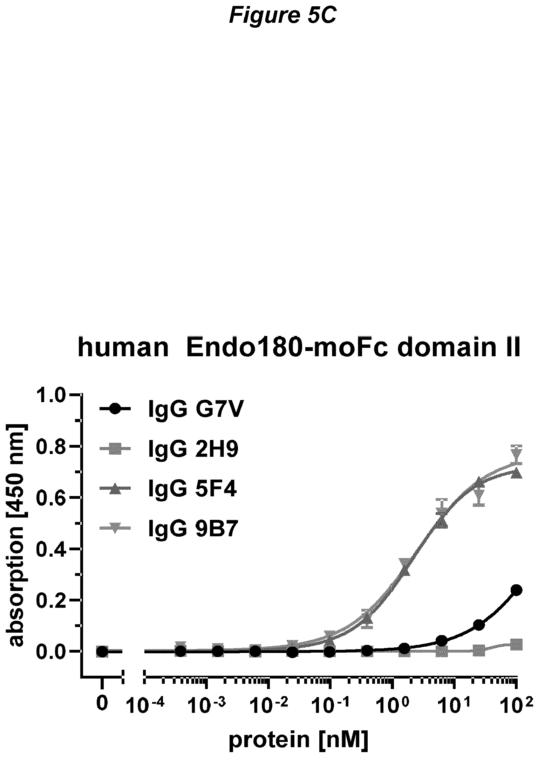

In a second ELISA experiment, the binding of the different anti-Endo180 antibodies to immobilized domain II of human Endo180 (3 µg/ml) was also tested. (Fig. 5). IgG 5F4 and IgG 9B7 showed a concentration-dependent binding with EC50 values of 2.2 nM and 2.5 nM, respectively. Marginal binding was detected for IgG G7V starting at a concentration of 25 nM. IgG 2H9 showed no binding to the domain II of human Endo180 (Figure 5C). Results are shown below in Table 3.

**Table 3: EC₅₀ values of anti-Endol80 antibodies to different domains of human Endl80-moFc molecules using ELISA experiments. n.d.: not determined; mean; n=1.**

| **huEndo180-moFc** | **DI-IV** | **DI-III** | **DI-II** | **DI** | **DII** |
|---|---|---|---|---|---|
| IgG G7V | 0.4 nM | 0.5 nM | n.d. | n.d. | n.d. |
| IgG 2H9 | 0.3 nM | 0.4 nM | 0.9 nM | 0.7 nM | n.d. |
| IgG 5F4 | 0.2 nM | 0.4 nM | 5.7 nM | n.d. | 2.2 nM |
| IgG 9B7 | 0.3 nM | 0.3 nM | 6.0 nM | n.d. | 2.5 nM |

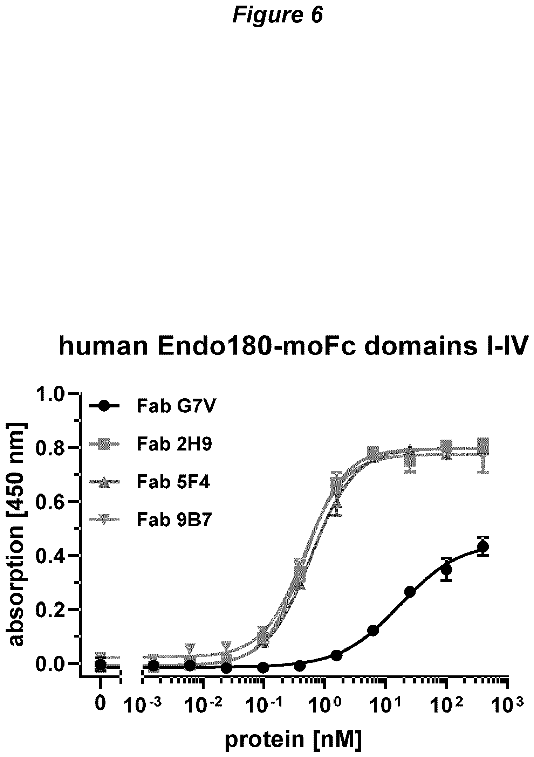

Based on these results, the monovalent Fab molecules of all anti-Endo180 antibodies were also generated to perform a competition analysis testing the binding of the Fab molecules to human Endo180 in the presence of pre-bound IgG G7V antibody. (Figure 6)

In the competition assay using ELISA experiment, human Endo180-moFc domains I-IV was used as immobilized antigen. The binding of all anti-Endo180 IgGs to human Endo180-moFc domains I-IV was detected in this experiment. In the case of the Fab molecules, only Fab 2H9, Fab 5F4 and Fab 9B7 showed a binding to human Endo180-moFc domains I-IV, while no binding was detected for Fab G7V molecule. For the competition assay, pre-incubating the immobilized human Endo180-moFc domains I-IV with 100 nM of IgG G7V, the competitor Fab molecules (Fab 2H9, Fab 5F4 and Fab 9B7) showed similar binding as observed for the Fab molecules without the pre-incubation of IgG G7V. However, no binding was detected for Fab G7V.

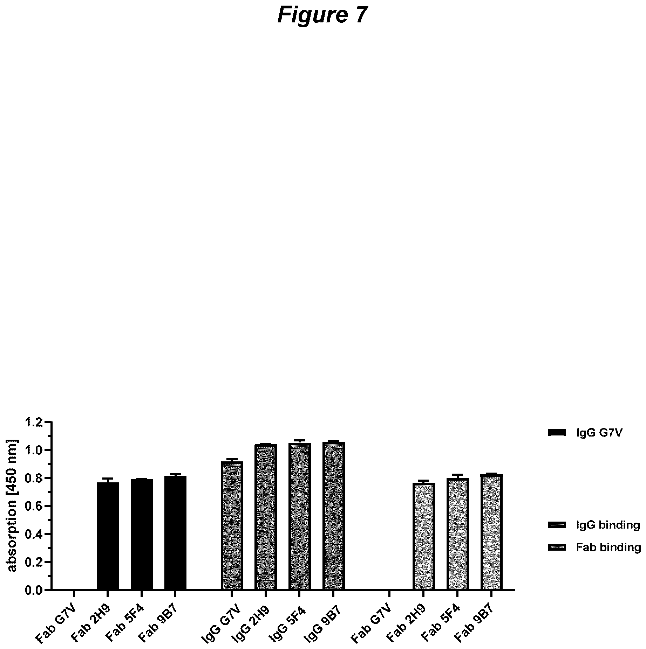

In the competition assay, all competitor anti-Endo180 Fab molecules (200 nM of Fab 2H9, Fab 5F4 and Fab 9B7) bound to human Endo180-moFc in the presence of bound IgG G7V molecule (100 nM), suggesting that the epitope of G7V is located on a different area compared to the competitor antibodies 2H9, 5F4 and 9B7. (Figure 7).

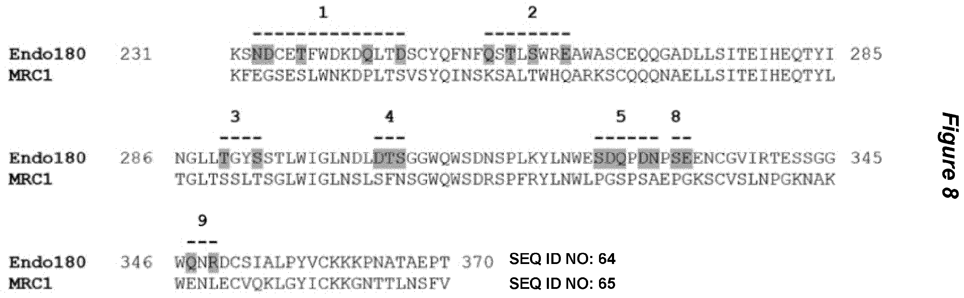

Since IgG G7V binds to domain III of human Endo180 molecule, human Endo180-moFc variants with different mutations in this domain were generated by substituting exposed residues within Endo180 domain III by those found in the structurally related MRC1 receptor, for further epitope mapping. In total, seven different regions of domain III of human Endo180 molecule (region 1, 2, 3, 4, 5, 8 and 9) were identified and cloned as DI-DIV-murine Fc fusion proteins (region 1: N233E, D234G, T237S, Q243P, D246S; region 2: Q254K, T256A, S258T, E261Q; region 3: T290S, S293T; region 4: D304S, T305F, S306N; region 5: S324P, D325G, Q326S, D328S, N3229A; region 8: S331A, E332G; region 9: Q347E, R349L) were generated (Figure 8). These variants were produced in HEK293 cells, purified by protein A chromatography and used as immobilized antigens in ELISA experiments tested the binding of 10 nM of IgG G7V and other anti-Endo180 antibodies (IgG 2H9, IgG 5F4 and IgG 9B7) in comparison to the wild-type version of human Endo180 molecule (DI-DIV).

In brief, the different mutated variants of human Endo180 were generated by gene synthesis and the cloned into expression plasmids for transfection into HEK293-6E cells. The produced regions expressed into the supernatant were purified with protein A affinity chromatography. For the ELISA experimNents, 3 µg/ml of the different variants and of the wild-type version of human Endo180 molecule were diluted in PBS and incubated with the ELISA plate overnight at 4°C. Afterwards, the plates were washed three times in PBS containing 0.005% (v/v) Tween 20 (PBST) and two times in PBS. The remaining binding sites were blocked with PBS, diluted with 2% (m/v) milk powder (MPBS) and incubated with the plate for 2 hours at room temperature. After washing the plates as described above, 10 nM of the different anti-Endo180 antibodies diluted in MPBS were added and detected with an HRP-conjugated anti-human Fc detection antibody (Sigma Aldrich; A01070; 1:5,000 diluted in MPBS) after washing the plates as described above. Finally, binding of the different antibodies was detected by using TMB/H2O2 after additional washing of the plates as described above. For IgG G7V binding to region 1, 2, 3, 4 and 9 did not show any differences compared to the wild-type version of human Endo180. However, strongly reduced binding was detected for IgG G7V to region 5 and 8. Thus, the epitope of IgG G7V is located within the region of human Endo180 from amino acid S324 to E332. The other regions (1, 2, 3, 4 and 9) are located on other parts of human Endo180 molecule. For the other anti-Endo180 antibodies IgG 2H9, 5F4 and 9B7 no differences were detected compared to the wild-type version of human Endo180 molecule, thus their epitopes are different from that of G7V.

To study their internalization efficiency, anti-Endo180 antibodies (IgG G7V, IgG 2H9, IgG 5F4 and IgG 9B7) were labeled with fluorophore (pHrodo, Sigma P36600), as described by the protocol of the manufacturer, and tested for internalization via flow cytometry, using the U2OS, T98G, SKOV3 and MDA-MB231 cell lines. In this experiment, 20 nM of the different pHrodo-labeled anti-Endo180 antibodies were added to the different cell lines and incubated for 5 minutes, 1 hour, 2 hours, 6 hours, 24 hours, and 48 hours at 37°C and for 5 minutes, 1 hour, 2 hours and 6 hours at 4°C.

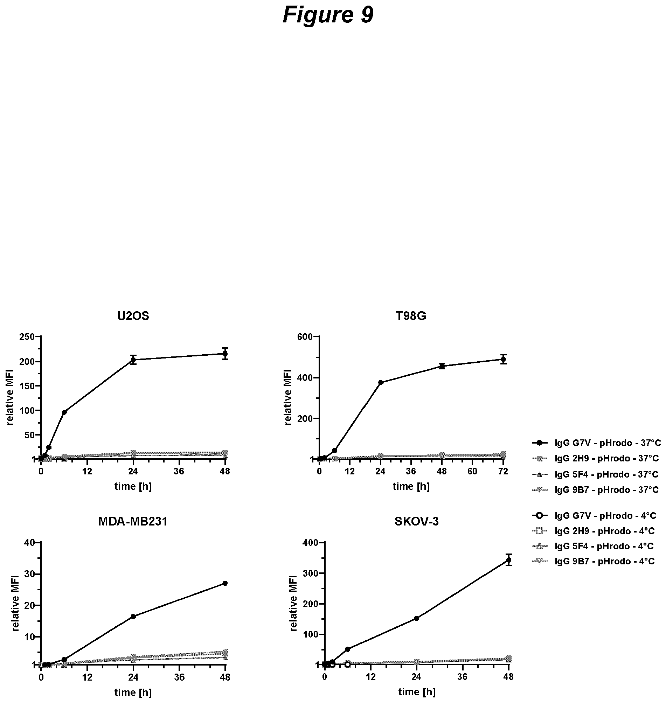

In general, the pHrodo-labeled anti-Endo180 antibodies showed a time-dependent internalization into the different cell lines. The fluorescence signal of U2OS, T98G, SKOV-3 and MDA-MB231 showed a higher fluorescence signal of pHrodo-labeled IgG G7V compared to the competitor antibodies. In U2OS and T98G, pHrodo-labeled IgG G7V internalization reached saturation after 24 hours, while for the other cell lines (SKOV-3, MDA-MB231), no saturation was evidenced even after 48 or 72 hours of incubation. (Figure 9)

Internalization of the different anti-Endo180 antibodies in U2OS, SKOV-3, MDA-MB231 and HEK293 cells, was also analyzed by incubating the cells at 4°C for up to 6 hours (Fig. 9). For pHrodo-labeled IgG G7V, while a strong internalization could be observed in U2OS and SKOV-3 cells incubated at 37°C for 6 hours, only reduced fluorescence intensity could be detected when incubating at 4°C. Similar results of reduced fluorescence intensity could be observed in MDA-MB231 and HEK293 cells.

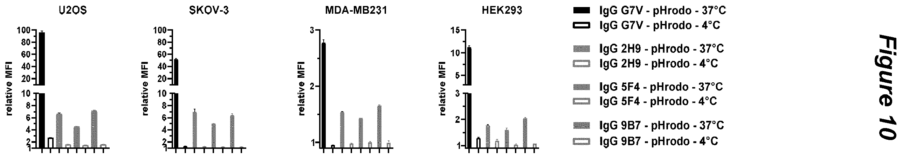

The other competitor antibodies IgG 2H9, IgG 5F4 and IgG 9B7 showed a reduced fluorescence intensity compared to IgG G7V in all cell lines incubated at 37°C for 6 hours. At 4°C, a reduced fluorescence intensity was obtained compared to the 37°C incubation. (Figure 10)

These results indicated that, although all anti-Endo180 antibodies are internalized at 37°C, G7V is the most efficiently internalized antibody in all the cell lines analyzed.

### EXAMPLE 3: Generation and production of anti-Endo 180 antibody drug conjugate

The purified G7V antibody was conjugated in Piramal at 500mg scale with the drug-linker TAM558 (cytolysin-linker maleimide caproyl vcPABA) to obtain OMTX707 antibody-drug conjugate.

The conjugation was a non-site directed cystein-based conjugation method through a thiol-based reaction.

The G7V antibody was reduced by 2.2 TCEP/mAb equivalents for 4 hours and conjugated with 6.0 TAM558/mAb equivalents for 1 hour. The conjugation was followed by a tangential flow filtration and formulation in 10 mM Sodium succinate, 6% w/v Sucrose, pH 5.5. The final protein was concentrated to 9.53mg/mL in formulation buffer.

Final OMTX707 ADC product obtained presented a drug-antibody ratio (DAR) of 3.79, with a 3.3% of free mAb content, analyzed by HIC-HPLC. The purity, analyzed by SEC-HPLC, was 96.9% of main peak, 0.5% of HMW and 2.6% of LMW. Free drug analyzed by RP-HPLC was 0.094% and endotoxin content was <1.55 EU/mL

For the obtention of OMTX807 ADC (antibody-drug conjugate), the purified G7V IgG antibody was conjugated by Wuxi at 500mg scale with the drug-linker GGFG-DXd. The conjugation method used was a non-specific site conjugation method on the cysteine residues of the antibody through a thiol-based reaction, similar as described for OMTX707.

The antibody was reduced by 9 TCEP/mAb equivalents for 2 hours and conjugated with 13 Dxd/mAb equivalents for 1 hour. The conjugation was followed by UFDF/Ultrafiltration/Charcoal purification and formulation in 20mM Histidine/HistidineHCl, 8% sucrose, pH 6.0. The final protein was concentrated to 12.30mg/mL in formulation buffer.

The final OMTX807 ADC product obtained presented a drug-antibody ratio (DAR) of 8.05 analyzed by HIC-HPLC and free mAb content of 3.3% analyzed by LC-MS. The purity was 97.29% of main peak and 2.71% of HMW. Free drug analyzed by HIC-HPLC was 1.18% and endotoxin content was <0.065 EU/mL.

### EXAMPLE 4: In vitro binding and activity of anti-Endo 180 antibody and conjugates

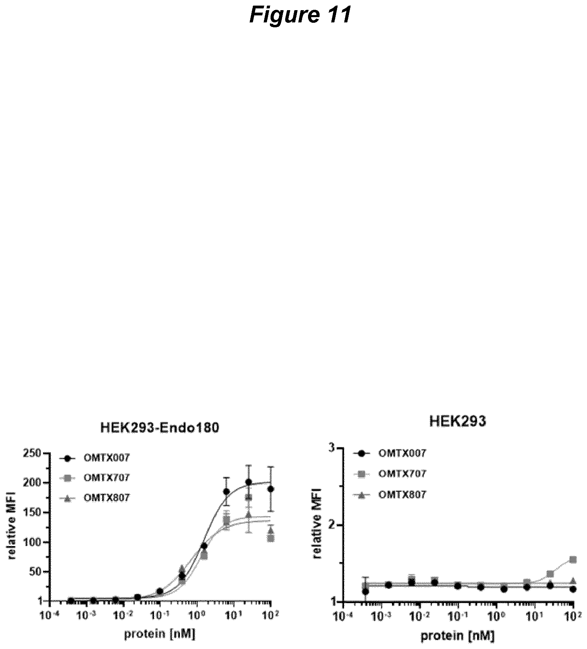

The different anti-Endo180 molecules OMTX007-G7V, OMTX707 and OMTX807 were tested for binding to HEK293 and HEK293-Endo180. All anti-Endo180 molecules (OMTX007-G7V, OMTX707 and OMTX807) showed a concentration-dependent binding to HEK293-Endo180 cells. For OMTX007-G7V and OMTX707, a saturated binding was detected for the Endo180-transfected cells HEK293-Endo180 with EC50 values of 1.5 nM for OMTX007-G7V and 1.3 nM for OMTX707. OMTX807 also showed a saturated binding to HEK293-Endo180 cells with slightly increased binding compared to OMTX007-G7V and OMTX707, with an EC50 value of 0.7 nM for HEK293-Endo180 cells. On the non-transfected parental HEK293 cells, no binding was detected in this assay for all analyzed anti-Endo180 antibodies, showing their Endo180 binding specificity. (Figure 11). Binding of OMTX007-G7V, OMTX707 and OMTX807 in HEK293-Endo180 as compared to non-transfected parental HEK293 cells is shown below in Table 4.

**Table 4: Summary of OMTX007-G7V, OMTX707 and OMTX807 binding on Endo180 versus parental HEK293 cells using flow cytometry analysis. EC50 values are shown; n.d.: not determined; mean: n=1.**

| | **OMTX007-G7V** | **OMTX707** | **OMTX807** |
|---|---|---|---|
| **HEK293-Endo180** | 1.5 nM | 1.3 nM | 0.7 nM |
| **HEK293** | n.d. | n.d. | n.d. |

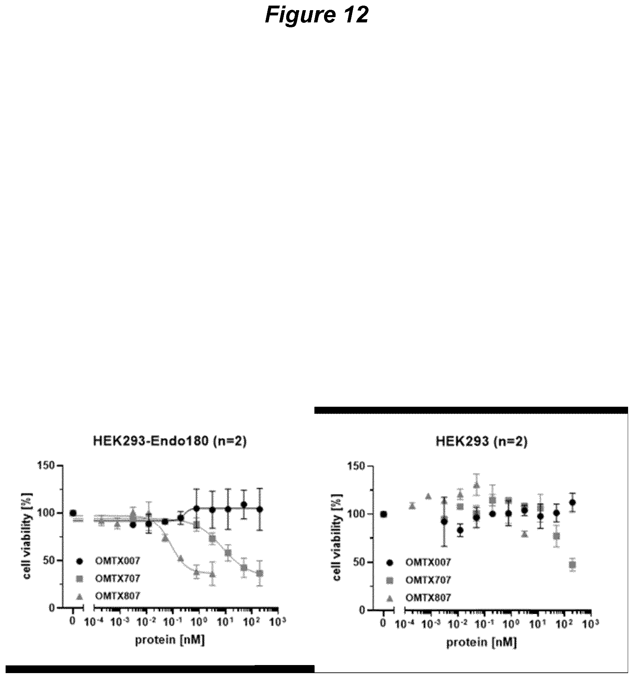

The different anti-Endo180 antibodies (OMTX007-G7V, OMTX707, OMTX807) were analyzed via cell viability assay using the HEK293-Endo180 and HEK293 cell lines. On HEK293-Endo180 cells, OMTX707 and OMTX807 showed a concentration-dependent killing with IC50 values of 83.9 ± 33.7 pM for OMTX807 and 7,523 ± 124 pM for OMTX707, thus stronger cell killing was detected for OMTX807 molecule compared to OMTX707, in correlation with their respective DAR value. No cell killing was detected with the unconjugated OMTX007 antibody and no IC50 values were reached up to 100nM for OMTX807 and OMTX707 in parental HEK293, showing that their activity was Endo180-dependent (see Table 5 below). (Figure 12).

**Table 5: Summary of OMTX007-G7V, OMTX707 and OMTX807 activity using cell viability analysis on Endo180 versus parental HEK293 cells. n.d.: not determined; mean ± SD: n=2.**

| | **OMTX007-G7V** | **OMTX707** | **OMTX807** |
|---|---|---|---|
| **HEK293-Endo180** | n.d. | 7,523 ± 124 pM | 83.9 ± 33.7 pM |
| **HEK293** | n.d. | n.d. | n.d. |

### EXAMPLE 5: Efficacy studies (OMTX807)

The antitumor activity of OMTX807 was evaluated in a IC9LC11 non-small cell lung cancer patient-derived xenograft model, developed in immunodeficient female mice.

OMTX807 was administered at 10 and 30mg/kg, both twice per week for 4 weeks. Docetaxel was also included in the study as the standard of care for this indication.

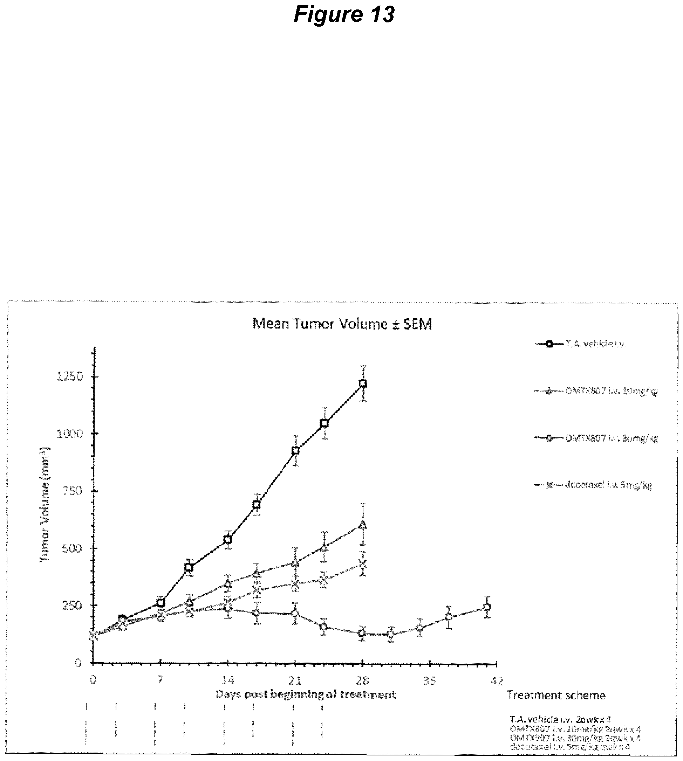

OMTX807 administered at 10 and 30 mg/kg demonstrated statistically significant antitumor efficacy with a dose-dependent effect (TGI=61% and 101% respectively at the control group end day, D28). (Figure 13)

Docetaxel administered at 5 mg/kg demonstrated statistically significant antitumor efficacy with TGI=73% at the control group end day, D28. (Figure 13)

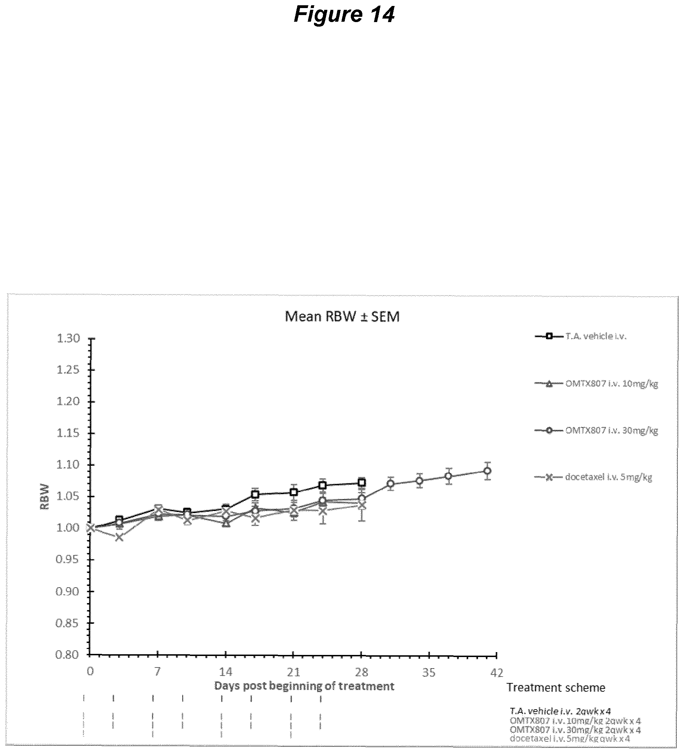

OMTX807 was well tolerated, based on body weight data and clinical observations. Docetaxel was also well tolerated, except in one mouse which showed a relative weight loss of 16.0% at the group end day. (Figure 14)

These results showed the superiority in terms of anti-tumoral effect of anti-Endo180 ADCs sharing Deruxtecan or derivatives cytotoxic payloads (OMTX807). OMTX807 also presented a higher TGI compared to the standard-of care Docetaxel. Furthermore, OMTX807 showed for the first time antitumoral efficacy of an anti Endo180 ADC in a solid tumor.

### EXAMPLE 6: Efficacy studies (OMTX707)

The antitumor activity of OMTX707 was evaluated in a subcutaneous SA4033 leiomyosarcoma patient-derived xenograft model developed in NOD/SCID immunodeficient female mice.

OMTX707 was administered intravenously at 10 and 30mg/kg once per week for 4 weeks.

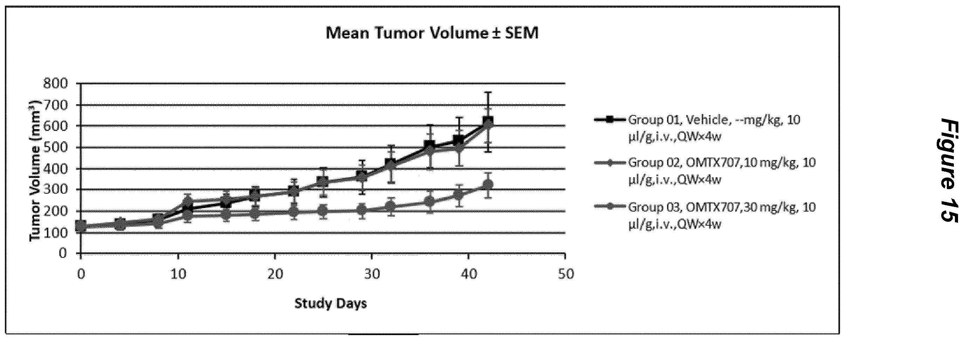

OMTX707 administered at 10 mg/kg once a week has no anti-tumor efficacy against SA4033 PDX model compared to the vehicle control group. OMTX707 administered at 30 mg/kg once a week produced a mild anti-tumor efficacy against SA4033 model, with TGI value of 48.10% on day 42, with no statistically significant difference (p=0.114), compared to vehicle control group (Figure 15).

OMTX707 administered at 10 and 30 mg/kg showed a dose-dependent effect (TGI=2.72% and 48.10% respectively to the control group on day 48) (Figure 15).

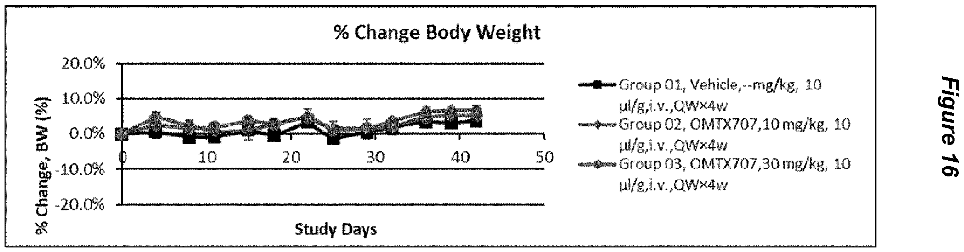

OMTX707 was well tolerated by mice, based on body weight data and clinical observations, with no adverse effects or animal death (Figure 16).

These results showed that OMTX707 at 30mg/kg against Endo180 target presents anti-tumor efficacy against leiomyosarcoma tumors.

### EXAMPLE 7: Affinity maturated G7V clone selection

Selections of G7V VH/VL phage libraries with randomized CDRs were performed. A list of clones with sequences and ELISA signals of the primary screen was provided and the top clones showing a signal above 1.2 were selected (see excel file). The clones were aligned with the sequence of G7V. Of note, more mutated positions were found in the VL sequences than the VH sequences.

An off-rate screening of scFvs (single chain fragment variables) was performed. Clones that did not show a reasonable off-rate were excluded from further characterization. The selected clones are shown below in Table 6.

**Table 6: G7V clones selected for further characterization**

| **Clone name** | **VH sequence** | **VL sequence** |
|---|---|---|
| **A11** | SEQ ID NO: 3 | SEQ ID NO: 6 |
| **A10** | SEQ ID NO: 3 | SEQ ID NO: 7 |
| **A04** | SEQ ID NO: 3 | SEQ ID NO: 8 |
| **H03** | SEQ ID NO: 3 | SEQ ID NO: 9 |
| **B03** | SEQ ID NO: 3 | SEQ ID NO: 10 |
| **E06** | SEQ ID NO: 3 | SEQ ID NO: 11 |
| **B02** | SEQ ID NO: 3 | SEQ ID NO: 12 |
| **D12** | SEQ ID NO: 3 | SEQ ID NO: 13 |
| **G10** | SEQ ID NO: 3 | SEQ ID NO: 14 |
| **C10** | SEQ ID NO: 4 | SEQ ID NO: 15 |

DNA encoding VH and VL of the selected clones was ordered and cloned into expression plasmids before being produced. Based on the off-rate data, the VH and VL of one additional clone C10 was also ordered.

Produced IgG and Fab molecules of the selected clones will be tested in ELISA for binding to recombinant Endo180 (D1-4, full-length, mouse Endo180 D1-4) and for binding to Endo180 expressing cells in comparison to the parental G7V and competitor antibodies. Affinity measurements will also be performed.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### References

A number of publications are cited above in order to more fully describe and disclose the invention and the state of the art to which the invention pertains. Full citations for these references are provided below. The entirety of each of these references is incorporated herein.
Curino, Alejandro C et al. "Intracellular collagen degradation mediated by uPARAP/Endo180 is a major pathway of extracellular matrix turnover during malignancy." The Journal of cell biology vol. 169,6 (2005): 977-85. Doi:10.1083/jcb.200411153
Gucciardo, Fabrice et al. "uPARAP/Endo180: a multifaceted protein of mesenchymal cells." Cellular and molecular life sciences : CMLS vol. 79,5 255. 22 Apr. 2022, doi:10.1007/s00018-022-04249-7
Nielsen, Christoffer Fagernaes et al. "The collagen receptor uPARAP/Endo180 as a novel target for antibody-drug conjugate mediated treatment of mesenchymal and leukemic cancers." Oncotarget vol. 8,27 (2017): 44605-44624. doi:10.18632/oncotarget.17883
Çak 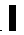 lkaya, P 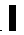 nar et al. "The Collagen Receptor uPARAP in Malignant Mesothelioma: A Potential Diagnostic Marker and Therapeutic Target." International journal of molecular sciences vol. 22,21 11452. 23 Oct. 2021, doi:10.3390/ijms222111452
Evans, Rachel J et al. "Endo180 (MRC2) Antibody-Drug Conjugate for the Treatment of Sarcoma." Molecular cancer therapeutics vol. 22,2 (2023): 240-253. Doi:10.1158/1535-7163.MCT-22-0312
For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

## Claims

1. A conjugate having the formula I:
A - (L-D)ₚ (I)
or a pharmaceutically acceptable salt or solvent thereof,
wherein:
A is an antibody that specifically binds domain III of Endo180;
L is a linker;
D is a drug comprising a topoisomerase inhibitor or a cytolysin; and
p is 1 to 20.

2. The conjugate of claim 1, wherein A exhibits a binding affinity dissociation constant K_{D} for domain III of Endo180 of less than 500 nM, optionally wherein A is a monoclonal antibody or binding fragment thereof that specifically binds to domain III of human and/or murine Endo180, further optionally wherein A binds an epitope located within the amino acid sequence SDQPDNPSE (SEQ ID NO: 63) of domain III of human Endo180.

3. The conjugate of claim 1 or 2, wherein A comprises heavy chain complementarity determining regions 1-3 (CDRH1-3) and light chain complementarity determining regions 1-3 (CDRL1-3) having the following amino acid sequences:
(i) CDRH1: GFTFX₁SYX₂MN;
(ii) CDRH2: NIKPX₃GX₄ERHSVX₅SVKG;
(iii) CDRH3: PGAGRLDY;
(iv) CDRL1: QGDX₆LRX₇X₈X₉AS;
(v) CDRL2: X₁₀X₁₁NX₁₂RPS; and
(vi) CDRL3: NSX₁₃X₁₄X₁₅X₁₆X₁₇X₁₈X₁₉X₂₀A;
wherein:
X₁ is S or P;
X₂ is S or A;
X₃ is D or N;
X₄ is S or N;
X₅ is D or E;
X₆ is S or R;
X₇ is S, R or G;
X₈ is Y, H, N, F or S;
X₉ is Y or F;
X₁₀ is G, F or V;
X₁₁ is K, R, or Q;
X₁₂ is N, K, G or S;
X₁₃ is R, L or P;
X₁₄ is D, G, N, or S;
X₁₅ is S, R, G or T;
X₁₆ is S or G;
X₁₇ is G, T, R or V;
X₁₈ is N, H, T or I;
X₁₉ is P or S; and
X₂₀ is W or P.

4. The conjugate of any one of claims 1-3, wherein CDRH1-3 comprise the following amino acid sequences:
(i) CDRH1: GFTFSSYSMN;
(ii) CDRH2: NIKPDGSERHSVDSVKG; and
(iii) CDRH3: PGAGRLDY,
optionally wherein CDRL1-3 comprise amino acid sequences selected from the group consisting of:
a.
(i) CDRL1: QGDSLRSYYAS;
(ii) CDRL2: GKNNRPS; and
(iii) CDRL3: NSRDSSGNPWA;
b.
(i) CDRL1: QGDSLRSHYAS;
(ii) CDRL2: FRNKRPS; and
(iii) CDRL3: NSRGSSGNPWA;
c.
(i) CDRL1: QGDSLRSNFAS;
(ii) CDRL2: VKNGRPS; and
(iii) CDRL3: NSRNSSTHPWA;
d.
(i) CDRL1: QGDRLRRNYAS;
(ii) CDRL2: GRNKRPS; and
(iii) CDRL3: NSRDRSGNPWA;
e.
(i) CDRL1: QGDSLRSFYAS;
(ii) CDRL2: GKNKRPS; and
(iii) CDRL3: NSLGSSGNPPA;
f.
(i) CDRL1: QGDRLRRNYAS;
(ii) CDRL2: GRNNRPS; and
(iii) CDRL3: NSRDGSGTPWA;
g.
(i) CDRL1: QGDSLRRSYAS;
(ii) CDRL2: FKNNRPS; and
(iii) CDRL3: NSRGTSRNPWA.
h.
(i) CDRL1: QGDSLRGYYAS;
(ii) CDRL2: GQNKRPS; and
(iii) CDRL3: NSRSRSRIPWA;
i.
(i) CDRL1: QGDSLRGYYAS;
(ii) CDRL2: GRNSRPS; and
(iii) CDRL3: NSRNRSGHPWA; or
j.
(i) CDRL1: QGDSLRSYYAS;
(ii) CDRL2: FKNNRPS; and
(iii) CDRL3: NSRGSSRNPWA.

5. The conjugate of any one of claims 1-5, wherein CDRH1-3 comprise having the following amino acid sequences:
(i) CDRH1: GFTFPSYAMN;
(ii) CDRH2: NIKPNGNERHSVESVKG; and
(iii) CDRH3: PGAGRLDY;
optionally wherein CDRL1-3 comprise the following amino acid sequences:
(i) CDRL1: QGDSLRSYFAS;
(ii) CDRL2: FKNNRPS; and
(iii) CDRL3: NSPNSGVISWA.

6. The conjugate of claim any one of the above claims, wherein A comprises:
a.
a heavy chain variable region (VH) comprising the amino acid sequence of SEQ ID NO: 3 or 4; and/or
a light chain variable region (VL) comprising an amino acid sequence selected from the group consisting of SEQ ID NO: 5-15.

7. The conjugate of any one of the above claims, wherein D is a topoisomerase inhibitor, optionally wherein the topoisomerase inhibitor is of formula II: 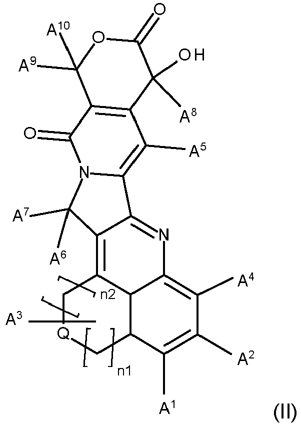 wherein:
A¹ and A² are each independently selected from the group consisting of halo, hydrogen, C₁-C₃ alkyl, phenyl, hydroxy, C₁-C₃ alkoxy,
or wherein A¹ and A² together with the atoms to which they are bonded form a fused ring;
Q is O, S or CR^{a}R^{b}; wherein R^{a} and R^{b} are each independently selected from hydrogen or a C₁-C₃ alkyl;
n₁ and n₂ are each individually 0, 1 or 2;
A³ is 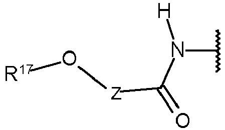 wherein
Z is a C₁-C₆ alkyl chain or a substituted or unsubstituted aryl group or wherein Z is X₁-Ar
wherein X₁ is a C₁-C₆ alkyl chain and Ar is a substituted or unsubstituted aryl group;
A⁴, A⁵, A⁶ A⁷, A⁹ and A¹⁰ are each individually hydrogen or a C₁-C₃ alkyl;
A⁸ is hydrogen or a C₁-C₄ alkyl;
R¹⁷ is indirectly or directly attached to linker L.

8. The conjugate of any one of claims 1-6, wherein D is a cytolysin, optionally wherein the cytolysin is of formula V: 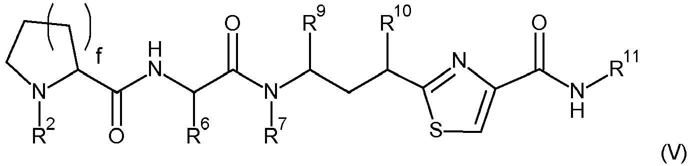 wherein:
R² is H or C₁-C₄ alkyl;
R⁶ is C₁-C₆ alkyl;
R⁷ is C₁-C₆ alkyl, CH₂OR¹⁹ or CH₂OCOR²⁰, wherein R¹⁹ is alkyl, R²⁰ is C₂-C₆-alkenyl, phenyl, or CH₂-phenyl;
R⁹ is C₁-C₆ alkyl;
R¹⁰ is O-alkyl, H, OH, or O-acetyl;
f is 2 or 1;
R¹¹ has the following structure: 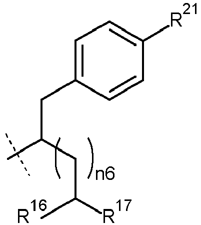 wherein
R²¹ is OH, H, halogen, NH₂, alkyloxy, phenyl, alkyl amino or dialkyl amino;
R¹⁶ is a C₁-C₆-alkyl or H;
R¹⁷ is indirectly or directly attached to linker L; and
n6 is 0, 1, 2 or 3;
wherein 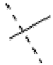 indicates the point of attachment to formula (V) and R¹¹.

9. The conjugate of claim 7 or 8, wherein R¹⁷ is C(O)X, CONHNHX, OX, NHX or SX, wherein X is a bond to linker L.

10. The conjugate of any of the above claims, wherein linker L further comprises a spacer, optionally wherein the spacer:
a. has a chain length of 2 to 30 atoms;
b. comprises or consists of an oxyalkylene or alkylene group;
c. comprises or consists of a group -(OCH₂CH₂)ₙ- or -(CH₂)ₙ-, wherein n = 1 to 15, 1 to 10, 2 to 9, 1 to 6, 2 to 5 or 3-5, optionally wherein n = 3 or 4;
d. is attached to group R¹⁷ via a bridging group or is directly attached to group R¹⁷; and/or
e. is attached to group R¹⁷ via a -C(O)X bridging group, wherein X is a bond to R¹⁷.

11. The conjugate of any of the above claims, wherein L comprises:
a. an attachment group for attachment to A and a protease cleavable portion;
b. a valine-citrulline unit; and/or
c. maleimidocaproyl-valine-citrulline-p-aminobenzylcarbamate,
optionally wherein the double bond of the maleimide is reacted with a thiol group of a cysteine residue of the antibody A to form a sulphur-carbon bond in order to effect linkage of the linker L to the antibody A.

12. The conjugate of any one of claims 7 and 9-11, wherein the topoisomerase inhibitor is of formula IV: 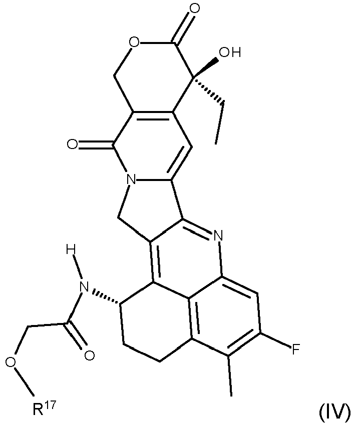 wherein R¹⁷ is indirectly or directly attached to linker L, optionally wherein -L-D has the following structure: 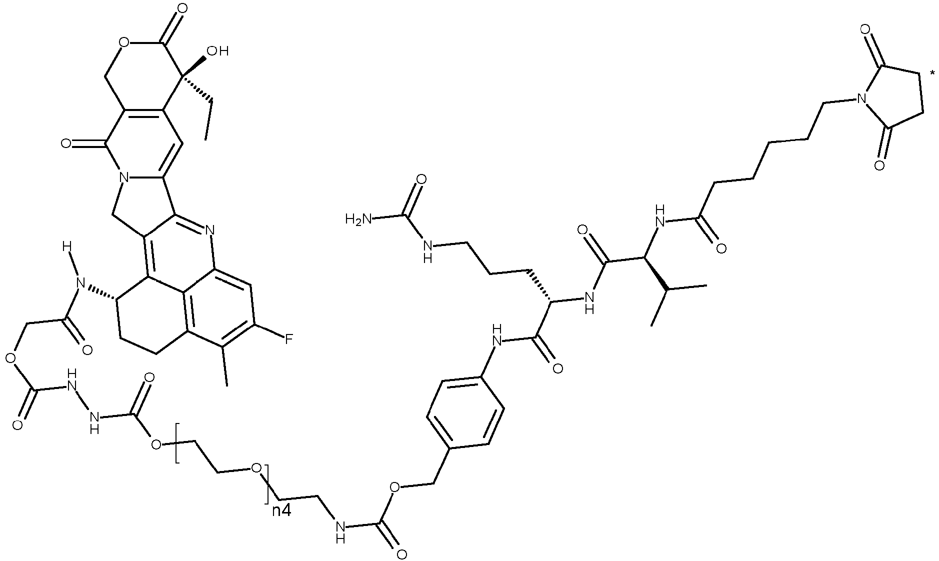 wherein n4 is 1 to 4 and * denotes the point of attachment to A, optionally wherein n4 is 3.

13. The conjugate of any one of claims 8-11, wherein the cytolysin is of formula VI: 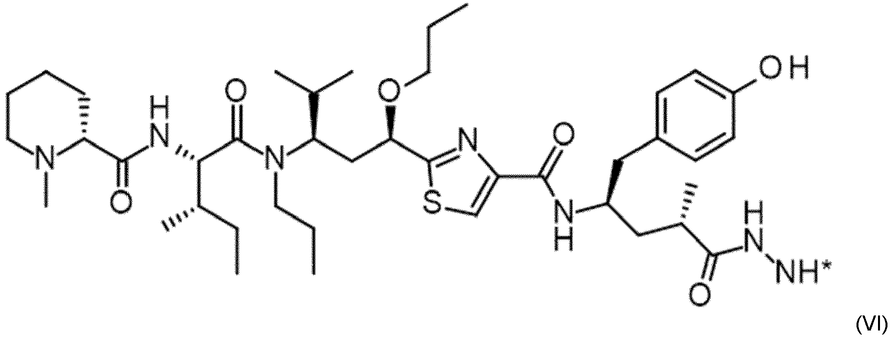 wherein * denotes the point of attachment to A, optionally wherein -L-D has the following structure: 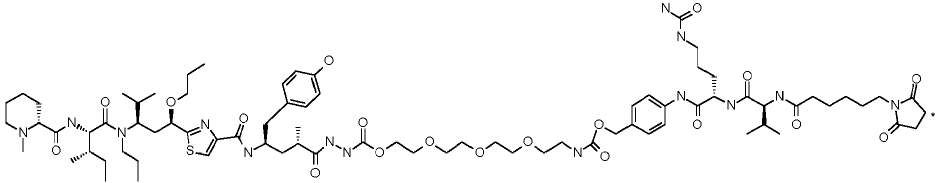 wherein * denotes the point of attachment to A.

14. A conjugate as defined in any of the above claims for use in a method of treatment of a cancer, fibrosis or an inflammatory disease in a mammalian subject, optionally wherein the cancer is a solid tumor cancer or wherein the inflammatory disease is arthritis.

15. A conjugate as defined in any of claims 7 and 9-12 for use in a method of treatment of a cancer in a mammalian subject, wherein the cancer is a carcinoma, optionally wherein the carcinoma is a non-small-cell lung cancer.

16. A conjugate as defined in any of claims 8-11 and 13 for use in a method of treatment of a cancer in a mammalian subject, wherein the cancer is a sarcoma, optionally wherein the sarcoma is a soft-tissue sarcoma, further optionally wherein the soft-tissue sarcoma is a leiomyosarcoma.

17. A process for the production of a conjugate as defined in any one of claims 1-13, comprising:
(a) linking the antibody that specifically binds domain III of Endo180 to the linker;
(b) linking the topoisomerase inhibitor or cytolysin to the linker; and
(c) optionally, purifying and/or isolating the conjugate, wherein steps (a) and (b) can be performed in any order.
